(19) Europäisches Patentamt / European Patent Office / Office européen des brevets

(11) **EP 4 068 291 A1**

(12) **EUROPEAN PATENT APPLICATION**
published in accordance with Art. 153(4) EPC

(43) Date of publication:
**05.10.2022 Bulletin 2022/40**

(21) Application number: **20894598.0**

(22) Date of filing: **27.11.2020**

(51) International Patent Classification (IPC):
**G16B 20/20** (2019.01)    **G16B 20/30** (2019.01)
**G16B 30/10** (2019.01)    **G16B 10/00** (2019.01)
**G16B 40/20** (2019.01)    **G06N 3/08** (2006.01)

(52) Cooperative Patent Classification (CPC):
**G06N 3/08; G16B 10/00; G16B 20/20; G16B 20/30; G16B 30/10; G16B 40/20**

(86) International application number:
**PCT/KR2020/017065**

(87) International publication number:
**WO 2021/107676 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.11.2019 KR 20190157257**

(71) Applicant: **GC Genome Corporation
Yongin-si, Gyeonggi-do 16924 (KR)**

(72) Inventors:
• **KI, Chang-Seok**
  **Yongin-si, Gyeonggi-do 16924 (KR)**
• **CHO, Eun Hae**
  **Yongin-si, Gyeonggi-do 16924 (KR)**
• **LEE, Junnam**
  **Yongin-si, Gyeonggi-do 16924 (KR)**
• **LEE, Tae-Rim**
  **Yongin-si, Gyeonggi-do 16924 (KR)**
• **AHN, Jin Mo**
  **Yongin-si, Gyeonggi-do 16924 (KR)**

(74) Representative: **Mewburn Ellis LLP
Aurora Building
Counterslip
Bristol BS1 6BX (GB)**

(54) **ARTIFICIAL INTELLIGENCE-BASED CHROMOSOMAL ABNORMALITY DETECTION METHOD**

(57) The present invention relates to an artificial intelligence-based chromosomal abnormality detection method, and more specifically, to an artificial intelligence-based chromosomal abnormality detection method using a method that involves: extracting nucleic acids from a biological sample to generate vectorized data on the basis of DNA fragments arranged by acquiring sequence information; and then comparing a reference value and a value calculated by inputting the vectorized data into a trained artificial intelligence model. Rather than using each of values related to reads as an individual normalized value as in existing schemes, which use a step for determining the amount of a chromosome on the basis of a read count, or existing detection methods using the distance concept between arranged reads, the artificial intelligence-based chromosomal abnormality detection method according to the present invention generates vectorized data and analyzes the data using an AI algorithm, and thus is useful in that a similar effect can be exhibited even when read coverage is low.

FIG. 1

```
┌─────────────────────────────────┐
│   Extracting cell-free nucleic acid   │
│           from plasma            │
└─────────────────────────────────┘
                 │
                 ▼
┌─────────────────────────────────┐
│  Obtaining nucleic acid fragments by  │
│     massive parallel sequencing    │
└─────────────────────────────────┘
                 │
                 ▼
┌─────────────────────────────────┐
│  Aligning nucleic acid fragment with  │
│   human reference genome database   │
└─────────────────────────────────┘
                 │
                 ▼
┌─────────────────────────────────┐
│    Determining quality of aligned data   │
└─────────────────────────────────┘
                 │
                 ▼
┌─────────────────────────────────┐
│  Data vectorization (i.e. GC plot, GCW plot)  │
└─────────────────────────────────┘
                 │
                 ▼
┌─────────────────────────────────┐
│    CNN (convolutional neural network)   │
└─────────────────────────────────┘
                 │
                 ▼
┌─────────────────────────────────┐
│  Calculation of deep probability (sigmoid)  │
│            index (DPI)            │
└─────────────────────────────────┘
                 │
                 ▼
┌─────────────────────────────────┐
│      Determining whether        │
│     aneuploidy is present       │
└─────────────────────────────────┘
```

**Description**

[Technical Field]

The present invention relates to a method for detecting a chromosomal abnormality based on artificial intelligence, and more specifically to a method for detecting a chromosomal abnormality based on artificial intelligence by extracting nucleic acids from a biological sample to obtain sequence information, generating vectorized data based on the aligned reads, and comparing a cut-off value with a calculated value obtained by inputting the vectorized data into a learned artificial intelligence model.

[Background Art]

Chromosomal abnormalities are associated with genetic defects and tumor-related diseases. The term "chromosomal abnormality" ma y mean deletion or duplication of chromosomes, deletion or duplication of a portion of chromosomes, or a break, translocation, or inversion in chromosomes. A chromosomal abnormality is a disorder related to genetic balance, and may cause fetal mortality or serious defects in physical and mental condition, as well as tumor-related diseases. For example, Down's syndrome is a common chromosome number abnormality caused by the presence of a third copy of chromosome 21 (which is also called "trisomy 21"). Edwards syndrome (trisomy 18), Patau syndrome (trisomy 13), Turner syndrome (XO), and Klinefelter syndrome (XXY) are also chromosomal abnormalities. Chromosomal abnormalities are also found in tumor patients. For example, duplication of chromosomes 4q, 11q, and 22q and deletion of chromosome 13q were observed in liver cancer patients (liver adenomas and adenocarcinomas patents), and duplication of chromosomes 2p, 2q, 6p, and 11q and deletion of chromosomes 6q, 8p, 9p, and 21 were observed in pancreatic cancer patients. These chromosomal regions are associated with tumor-related oncogene and tumor suppressor gene regions.

Chromosomal abnormalities can be detected using karyotype and FISH (fluorescent in-situ hybridization). This detection method is disadvantageous in terms of time, effort, and accuracy. In addition, DNA microarrays can be used to detect chromosomal abnormalities. In particular, a genomic DNA microarray system is capable of easily producing a probe and detecting chromosomal abnormalities in the intron region of the chromosome as well as the extended region of the chromosome, but is difficult to use to produce a large number of DNA fragments, the chromosomal locations and functions of which have been identified.

Recently, next-generation sequencing has been used to analyze chromosome number abnormalities (Park, H., Kim et al., Nat Genet 2010, 42, 400-405.; Kidd, J.M. et al., Nature 2008, 453, 56-64). However, this technology requires high-coverage reading for the analysis of chromosome number abnormalities (aneuploidy), and CNV measurements also require independent validation. Therefore, this technology has been unsuitable for general gene search analysis in the prior art because it is expensive and the results thereof are difficult to understand.

Meanwhile, conventional prenatal test items for fetal chromosomal abnormalities include ultrasound examination, blood marker tests, amniocentesis, chorionic examination, transdermal umbilical cord blood tests, and the like (Mujezinovic F. et al., Obstet. Gynecol. 2007, 110(3):687-94.). Among them, ultrasound examination and blood marker tests are classified as screening tests, and amniocentesis is classified as a confirmatory test. Ultrasound examination and blood marker tests, which are non-invasive methods, are safe because they do not involve directly collecting samples from the fetus, but the sensitivity of the tests is low, specifically 80% or less (ACOG Committee on Practice Bulletins. 2007). Invasive methods such as amniocentesis, chorionic examination, and transdermal umbilical cord blood tests are capable of detecting fetal chromosomal abnormalities, but have a disadvantage in that there is the possibility of loss of the fetus due to the invasive nature of the medical procedure.

Lo et al. succeeded in sequencing the Y chromosome of a fetal genetic material from maternal plasma and serum in 1997 and then used the fetal genetic material derived from the mother for prenatal testing (Lo Y. M., et al. The Lancet. 1997, 350 (9076) :485-7) . The fetal genetic material that is present in the maternal blood is some trophoblast cells that undergo apoptosis during placental remodeling and enter the maternal blood through a substance exchange mechanism, is actually derived from the placenta, and is defined as "cff DNA (cell-free fetal DNA)".

cff DNA is detected as early as day 18 of embryo transfer and is found in most maternal blood at day 37 of embryo transfer. cff DNA is a short strand of 300 bp or less and is present in a small amount in maternal blood, so massive parallel sequencing technology using next-generation sequencing (NGS) is used to apply the same to the detection of fetal chromosomal abnormalities. Non-invasive fetal chromosomal abnormality detection using massive parallel sequencing has detection sensitivity of 90-99% or more depending on the chromosome, but causes false-positive and false-negative results in 1 to 10% of cases, and thus requires correction therefor (Gil MM, et al. Ultrasound Obstet. Gynecol. 2015, 45(3):249-66).

Meanwhile, research is actively being conducted on the practical application of the inherent and efficient pattern recognition capability of humans to computers as a solution to the issue of classifying input patterns into specific groups, which is common in the engineering field.

Among various studies relating to application of computers thereto, a study has been performed on the artificial neural network that is obtained by modeling the cellular structure of the human brain, in which efficient pattern recognition occurs, in an engineering manner.

To solve the issue of classification of input patterns into specific groups, artificial neural networks use algorithms that mimic the learning ability of humans. Based on such an algorithm, artificial neural networks create mapping between the input patterns and the output patterns, so it can be said that artificial neural networks have the ability to learn. In addition, artificial neural networks are capable of performing generalization based on learning results to create the most appropriate outputs in response to input patterns that were not used for learning based on learning results. Due to the two representative performance of learning and generalization, artificial neural networks are being applied to problems that are difficult to solve with conventional sequential programming methods. Artificial neural networks have a wide range of applicability and thus are actively applied to fields of pattern classification problems, continuous mapping, non-linear system identification, non-linear control, robot control, and the like.

Artificial neural networks are computational models implemented in software or hardware that mimic the computational ability of biological systems using a large number of artificial neurons connected via connective lines. Artificial neural networks use artificial neurons, which represent the functions of biological neurons in simplified form. Artificial neural networks conduct human cognition or learning processes by interconnecting the artificial neurons through connective lines having respective connection intensities. The term "connection intensity", which is interchangeable with "connection weight, refers to a predetermined value of the connection line.

Artificial neural network learning may be classified into supervised learning and unsupervised learning. Supervised learning is a method of providing input data and output data corresponding thereto to a neural network and updating the connection intensities of connecting lines so that output data corresponding to the input data is output. Representative learning algorithms include delta rule and back propagation learning. Unsupervised learning is a method in which an artificial neural network independently learns connection intensities using only input data, without a target value. Unsupervised learning updates connection weights based on correlations between input patterns.

Applying large amounts of data to machine learning causes the so-called "curse of dimensionality" problem due to the increased complexity and the greater number of dimensions. In other words, as the number of dimensions of the required data approaches infinity, the distance between any two points also approaches infinity, and the amount of data, that is, the density, becomes lower in high-dimensional space, which makes it impossible to properly reflect the features of the data (Richard Bellman, Dynamic Programming, 2003, chapter 1). Recently developed deep learning has a structure in which a hidden layer is present between an input layer and an output layer, and was reported to greatly improve the performance of the classifier in high-dimensional data such as images, videos, and signal data by processing a linear combination of variable valuestransmitted from the input layer with nonlinear functions (Hinton, Geoffrey, et al., IEEE Signal Processing Magazine Vol. 29.6, pp. 82- 97, 2012).

Various patents (KR 10-2017-0185041, KR 10-2017-0144237, KR 10-2018-124550) describe the use of artificial neural networks in biological fields, but there is a lack of research on methods for detecting chromosomal abnormalities through artificial neural network analysis based on cffDNA sequencing information.

Accordingly, as a result of extensive and earnest efforts to solve the above problems and develop a method for detecting chromosomal abnormalities based on artificial intelligence with high sensitivity and accuracy, the present inventors found that chromosomal abnormalities can be detected with high sensitivity and accuracy by generating vectorized data based on reads aligned in chromosomal regions and analyzing the data using a trained artificial intelligence model, and the present invention has been completed based on this finding.

[Disclosure]

Therefore, it is one object of the present invention to provide a method for detecting chromosomal abnormalities based on artificial intelligence.

It is another object of the present invention to provide a device for determining a chromosomal abnormality based on artificial intelligence.

It is another object of the present invention to provide a computer-readable storage medium including instructions configured to be executed by a processor for determining a chromosomal abnormality by the method described above.

In accordance with one aspect of the present invention, the above and other objects can be accomplished by the provision of a method of detecting a chromosomal abnormality based on artificial intelligence, the method including a) obtaining sequence information using extracted nucleic acids from a biological sample, b) aligning the sequence information (reads) with a reference genome database, c) generating vectorized data based on the aligned sequence information (reads), and d) inputting the generated vectorized data into a trained artificial intelligence model, analyzing the data, and comparing the resulting value with a cut-off value to determine whether there is a chromosomal abnormality.

In accordance with another aspect of the present invention, provided is a device for determining a chromosomal abnormality based on artificial intelligence, the device including a decoder configured to extract nucleic acids from a biological sample and decode sequence information, an aligner configured to align the decoded sequence with a reference genome database, a data generator configured to generate vectorized data based on aligned sequence information (reads), and a chromosomal abnormality determiner configured to input the generated vectorized data into a trained artificial intelligence model, analyze the data, and compare the resulting value with a cut-off value thereby to determine whether or not a chromosomal abnormality is present.

In accordance with another aspect of the present invention, provided is a computer-readable storage medium including an instruction configured to be executed by a processor for detecting a chromosomal abnormality through the following steps including a) obtaining sequence information using extracted nucleic acids from a biological sample, b) aligning the obtained sequence information (reads) with a reference genome database, c) generating vectorized data based on the aligned sequence information (reads), and d) inputting the generated vectorized data to a trained artificial intelligence model, analyzing the data, and comparing the resulting value with a cut-off value to determine whether or not a chromosomal abnormality is present.

[Description of Drawings]

FIG. 1 is an overall flowchart for determining a chromosomal abnormality based on artificial intelligence according to the present invention.

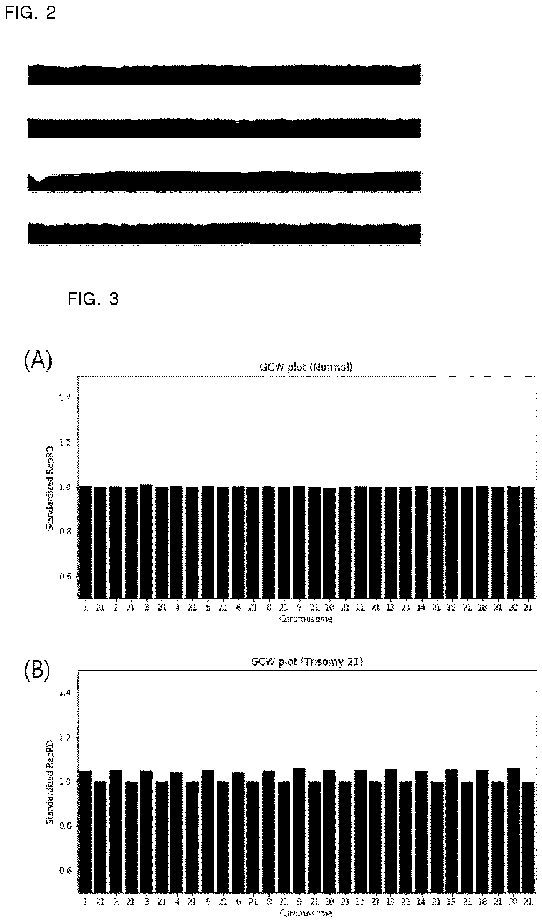

FIG. 2 is an example of a GC plot generated according to an embodiment of the present invention, wherein the X-axis represents a chromosome for each bin and the Y-axis represents a distance between nucleic acid fragments in each bin.

FIG. 3 shows (A) an example of a GCW plot of a normal sample generated according to an embodiment of the present invention and (B) an example of a GCW plot of a sample having three chromosomes 21, wherein the X-axis represents the chromosome, and the Y-axis represents a Z score, converted from the distance between the nucleic acid fragments corresponding to the chromosome.

FIG. 4 shows (A) an example of a GCW plot of a normal sample generated according to an embodiment of the present invention and (B) an example of a GCW plot of a sample having three chromosomes 21, wherein the X-axis represents the chromosome and the Y-axis represents a Z score, converted from the number of reads corresponding to the chromosome.

FIG. 5 shows (A) the accuracy of trisomy 21 detection for a deep-learning model that had learned GC plot image data generated based on the distance between the nucleic acid fragments according to an embodiment of the present invention, and (B) the probability distribution for each data set.

FIG. 6 shows (A) the accuracy of trisomy 18 detection for a deep-learning model that had learned the GC plot image data generated based on the distance between the nucleic acid fragments according to an embodiment of the present invention, and (B) the probability distribution for each data set.

FIG. 7 shows (A) the accuracy of trisomy 13 detection for a deep-learning model that had learned the GC plot image data generated based on the distance between the nucleic acid fragments according to an embodiment of the present invention, and (B) the probability distribution for each data set.

FIG. 8 shows the accuracy of trisomy 21 detection for a deep-learning model that had learned the GCW plot image data generated based on the distance between the nucleic acid fragments according to an embodiment of the present invention (upper panel), and the probability distribution for each data set (lower panel).

FIG. 9 shows the accuracy of the result of detection of the abnormality of chromosome 18 by the trained deep-learning model in FIG. 8 (left panel), and the probability distribution (right panel).

FIG. 10 shows the accuracy of the result of detection of the abnormality of chromosome 13 by the trained deep-learning model in FIG. 8 (left panel), and the probability distribution (right panel).

FIG. 11 shows the accuracy of downsampling to detect the abnormality of chromosome 21 by the trained deep-learning model in FIG. 8 (left panel), and the probability distribution (right panel).

FIG. 12 shows the accuracy of trisomy 21 detection for a deep-learning model that had learned GCW plot image data generated based on the reciprocal of the distance between the nucleic acid fragments according to an embodiment of the present invention (upper panel) and the probability distribution for each data set (lower panel).

FIG. 13 shows the accuracy of the result of detection of the abnormality of chromosome 18 by the trained deep-learning model in FIG. 12 (upper panel) and the probability distribution (right panel).

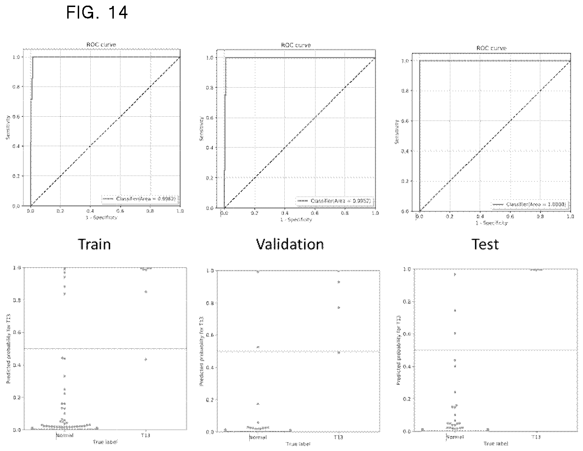

FIG. 14 shows the accuracy of the result of detection of the abnormality of chromosome 13 by the trained deep-learning model in FIG. 12 (upper panel), and the probability distribution (right panel).

[Best Mode]

Unless defined otherwise, all technical and scientific terms used herein have the same meanings as appreciated by those skilled in the field to which the present invention pertains. In general, the nomenclature used herein is well-known in the art and is ordinarily used.

It was found in the present invention that a chromosomal abnormality can be detected with high sensitivity and accuracy by aligning sequencing data obtained from a sample with a reference genome, generating vectorized data based on the aligned nucleic acid fragments, calculating a DPI using a trained artificial intelligence model, and comparing the DPI with a cut-off value to determine whether there is a chromosomal abnormality.

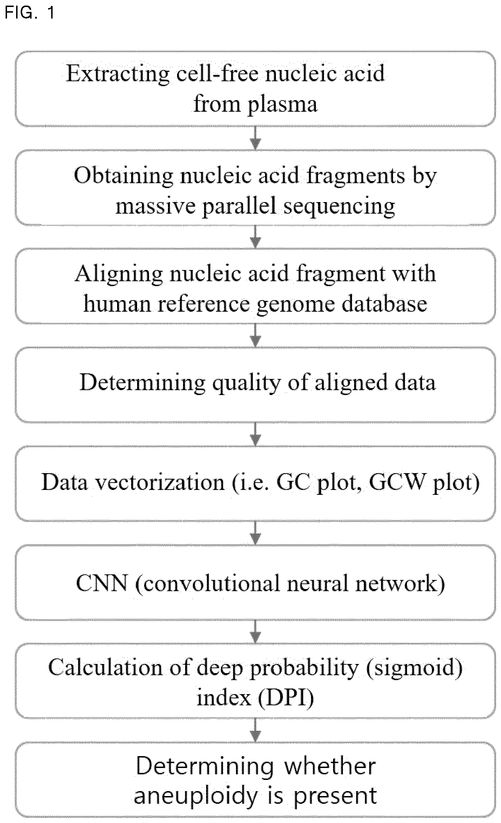

That is, in one embodiment of the present invention, developed is a method including sequencing DNA extracted from blood, aligning the sequencing data with a reference genome, calculating the distance between nucleic acid fragments or amount of the nucleic acid fragments in each predetermined chromosomal bin, generating vectorized data with the chromosomal bin on the X-axis and the distance between nucleic acid fragments or amount thereof on the Y-axis, allowing a deep-learning model to perform learning on the data to calculate a DPI and, determining that there is a chromosomal abnormality when the DPI is equal to or greater than a cut-off value (FIG. 1).

In one aspect, the present invention is directed to a method of detecting a chromosomal abnormality based on artificial intelligence, the method including:

a) obtaining sequence information using extracted nucleic acids from a biological sample;

b) aligning the sequence information (reads) with a reference genome database;

c) generating vectorized data based on the aligned nucleic acid fragments; and

d) inputting the generated vectorized data into a trained artificial intelligence model, analyzing the data, and comparing the resulting value with a cut-off value to determine whether or not there is a chromosomal abnormality.

In the present invention, any nucleic acid fragment can be used without limitation, as long as it is a fragment of a nucleic acid extracted from a biological sample, and the nucleic acid fragment is preferably a fragment of a cell-free nucleic acid or an intracellular nucleic acid, but is not limited thereto.

In the present invention, the nucleic acid fragment may be obtained by direct sequencing, next-generation sequencing, or sequencing through nonspecific whole genome amplification.

In the present invention, the nucleic acid fragment may mean a read when next-generation sequencing is used.

As used herein, the term "chromosomal abnormality" is defined to include a variety of variations occurring in chromosomes, and may be broadly divided into number abnormalities, structural abnormalities, microdeletions, chromosomal instability, and the like.

"Chromosome number abnormality" refers to the case in which an abnormality occurs in the number of chromosomes, and may include all cases of deviation from a total of 46 chromosomes in 23 pairs, such as Down's syndrome (in which the total number of chromosomes is 47, including one extra copy of chromosome 21), Turner syndrome (in which the total number of chromosomes is 45 due to the presence of only one X chromosome), and Klinefelter syndrome (having an abnormal number of chromosomes, such as XXYY, XXXY, and XXXXY).

Structural chromosomal abnormalities refer to all cases in which there is no change in the number of chromosomes but there is a change in the structure of chromosomes, such as deletions, duplications, inversions, and translocations. Examples thereof include partial deletion of chromosome 5 (cat's cry syndrome), partial deletion of chromosome 7 (Phillips syndrome), partial duplication of chromosome 12 (Wolf-Hirschhorn syndrome), translocation between chromosomes 9 and 22 (chronic myelogenous leukemia), and the like. Examples also include microduplication and microdeletion of some chromosomes found in tumor patients, but the invention is not limited thereto.

In the present invention,

step (a) includes:

(a-i) obtaining nucleic acids from the blood, semen, vaginal cells, hair, saliva, urine, oral cells, amniotic fluid containing placental cells or fetal cells, tissue cells, and a mixture thereof;

(a-ii) obtaining purified nucleic acids by removing proteins, fats, and other residues from the obtained nucleic acids using a salting-out method, a column chromatography method, or a bead method;

(a-iii) preparing a single-end sequencing or paired-end sequencing library for the purified nucleic acids or nucleic acids randomly fragmented by an enzymatic digestion, pulverization, or hydroshear method;

(a-iv) reacting the prepared library with a next-generation sequencer; and

(a-v) obtaining sequence information (reads) of the nucleic acids in the next-generation sequencer.

In the present invention, the next-generation sequencer may be used for any sequencing method known in the art. Sequencing of nucleic acids isolated using the selection method is typically performed using next-generation sequencing (NGS). Next-generation sequencing includes any sequencing method that determines the nucleotide sequence either of each nucleic acid molecule or of a proxy cloned from each nucleic acid molecule so as to be highly similar thereto (e.g., $10^5$ or more molecules are sequenced simultaneously). In one embodiment, the relative abundance of nucleic acid species in the library can be estimated by counting the relative number of occurrences of the sequence homologous thereto in data produced by sequencing experimentation. Next-generation sequencing is known in the art, and is described, for example, in Metzker, M. (2010), Nature Biotechnology Reviews 11:31-46, which is incorporated herein by reference.

In one embodiment, next-generation sequencing is performed to determine the nucleotide sequence of each nucleic acid molecule (using, for example, a HelioScope Gene-Sequencing system from Helicos Biosciences or a PacBio RS system from Pacific Biosciences). In other embodiments, massive parallel short-read sequencing, which produces more bases of the sequence per sequencing unit than other sequencing methods, for example, other sequencing methods that produce fewer but longer reads, determines the nucleotide sequence of a proxy cloned from each nucleic acid molecule (using, for example, a Solexa sequencer from Illumina Inc., located in San Diego, CA; 454 Life Sciences (Branford, Connecticut) and Ion Torrent). Other methods or devices for next-generation sequencing may be provided by 454 Life Sciences (Branford, Connecticut), Applied Biosystems (Foster City, CA; SOLiD Sequencer), Helicos Biosciences Corporation (Cambridge, MA) and emulsion and microfluidic sequencing nanodrops (e.g., GnuBIO Drops), but are not limited thereto.

Platforms for next-generation sequencing include, but are not limited to, the FLX System genome sequencer (GS) from Roche/454, the Illumina/Solexa genome analyzer (GA), the Support Oligonucleotide Ligation Detection (SOLiD) system from Life/APG, the G.007 system from Polonator, the HelioScope gene-sequencing system from Helicos Biosciences, and the PacBio RS system from Pacific Biosciences.

NGS technologies may, for example, include one or more of template production, sequencing, imaging, and data analysis steps.

Template production. Methods for producing templates include randomly disrupting nucleic acids (e.g., genomic DNA or cDNA) into small sizes and producing sequencing templates (e.g., fragment templates or mate-pair templates). Spatially separated templates may be attached or immobilized on a solid surface or support, which allows simultaneous large-scale sequencing reactions to be performed. Examples of types of templates that can be used for NGS reactions include templates amplified from clones derived from single DNA molecules and single DNA molecule templates.

Methods for producing the templates amplified from clones include, for example, emulsion PCR (emPCR) and solid-phase amplification.

EmPCR may be used to produce templates for NGS. Typically, a library of nucleic acid fragments is produced, and adapters containing universal priming sites are ligated to the ends of the fragments. The fragments are then denatured into single strands and captured using beads. Each bead captures a single nucleic acid molecule. After amplification and enrichment of emPCR beads, a large amount of templates can be attached, immobilized to a polyacrylamide gel on a standard microscope slide (from, for example, Polonator) and chemically crosslinked to an amino-coated glass surface (e.g., Life/APG; Polonator), or deposited in individual PicoTiterPlate (PTP) wells (e.g., Roche/454). At this time, an NGS reaction may be performed.

Solid-phase amplification can also be used to produce templates for NGS. Typically, the front and rear primers are covalently attached to the solid support. The surface density of the amplified fragment is defined as the ratio of primer to template on the support. Solid-phase amplification is capable of producing millions of spatially separated template clusters (e.g., Illumina/Solexa). The ends of the template cluster can be hybridized to universal primers for NGS reactions.

Other methods for producing clone-amplified templates include, for example, multiple displacement amplification (MDA) (Lasken R. S.; Curr. Opin. Microbiol. 2007; 10(5):510-6). MDA is a non-PCR-based DNA amplification method. The reaction involves annealing random hexamer primers to templates and synthesizing DNA using a high-fidelity enzyme, typically $\Phi$29, at a constant temperature. MDA can yield large-scale products with a lower error frequency.

Template amplification methods such as PCR can bind the NGS platform to the target or enrich specific regions of the genome (e.g., exons). Representative template enrichment methods include, for example, microdroplet PCR (Tewhey R. et al., Nature Biotech. 2009, 27:1025-1031), custom-designed oligonucleotide microarrays (e.g., Roche/NimbleGen oligonucleotide microarrays), solution-based hybridization (e.g., molecular inversion probes, MIPs) (Porreca GJ et al., Nature Methods, 2007, 4:931-936; Krishnakumar S. et al., Proc. Natl. Acad. Sci. USA, 2008, 105:9296-9310; Turner EH et al., Nature Methods, 2009, 6:315-316), and biotinylated RNA capture sequences (Gnirke A. et al., Nat. Biotechnol. 2009;27(2):182-9).

Single-molecule templates are another type of template that can be used for NGS reactions. Spatially separated single-molecule templates may be immobilized on a solid support by a variety of methods. In one approach, each primer molecule is covalently attached to a solid support. The adapter is added to the template and the template is then hybridized to the immobilized primer. In another approach, a single-molecule template is covalently attached to a solid support by priming and extending a single-stranded single-molecule template from the immobilized primer. The universal primer is then hybridized to the template. In another approach, a single polymerase molecule is attached to a solid support to which a primed template is bound.

Sequencing and imaging. Representative sequencing and imaging methods for NGS include, but are not limited to, cyclic reversible termination (CRT), sequencing by ligation (SBL), pyrosequencing, and real-time sequencing.

CRT uses reversible terminators in a cyclic method that includes, at a minimum, steps of nucleotide incorporation, fluorescence imaging, and cleavage. Typically, a DNA polymerase incorporates a single fluorescently modified nucleotide complementary to the nucleotide of the template base in the primer. DNA synthesis is terminated after incorporation of a single nucleotide, and the unincorporated nucleotides are washed out. Imaging is performed to determine the homology of the incorporated labeled nucleotides. Then, in the cleavage step, the terminator/inhibitor and the fluorescent dye are removed. Representative NGS platforms using the CRT method include, but are not limited to, Illumina/Solexa Genome Analyzer (GA), which uses a clone-amplification template method combined with a 4-color CRT method involving detection using total internal reflection fluorescence (TIRF); and Helicos Biosciences/HelioScope, using a single-molecule template method combined with a 1-color CRT method involving detection using TIRF.

SBL uses a DNA ligase and either a 1-base-encoded probe or a 2-base-encoded probe for sequencing.

Typically, a fluorescently labeled probe is hybridized to a complementary sequence adjacent to the primed template. DNA ligases are used to ligate dye-labeled probes to primers. After the non-ligated probes are washed, fluorescence imaging is performed to determine the identity of the ligated probes. The fluorescent dye may be removed using a cleavable probe that regenerates the 5'-PO4 group for subsequent ligation cycles. Alternatively, new primers may be hybridized to the template after old primers have been removed. Representative SBL platforms include, but are not limited to, Life/APG/SOLiD (support oligonucleotide ligation detection), which uses a two-base-encoded probe.

The pyrosequencing method is based on detection of activity of DNA polymerase with another chemiluminescent enzyme. Typically, this method includes sequencing a single strand of DNA by synthesizing complementary strands of one base pair at a time and detecting the base that is actually added at each step. The template DNA is stationary, and solutions of A, C, G, and T nucleotides are sequentially added and removed during the reaction. Light is generated only when the nucleotide solution replenishes the unpaired base of the template. The sequence of the solution generating the chemiluminescent signal is used to determine the sequence of the template. Representative pyrosequencing platforms include, but are not limited to, those from Roche/454, using DNA templates produced from 1 to 2 million beads deposited in PTP wells by emPCR.

Real-time sequencing involves imaging the continuous incorporation of dye-labeled nucleotides during DNA synthesis. Representative real-time sequencing platforms include, but are not limited to, a platform from Pacific Biosciences, which uses DNA polymerase molecules attached to the surface of respective zero-mode waveguide (ZMW) detectors to obtain sequence information when phosphate-linked nucleotides are incorporated in the growing primer strands; the Life/VisiGen platform using genetically engineered DNA polymerases along with attached fluorescent dyes to create an enhanced signal after incorporation of the nucleotide by fluorescence resonance energy transfer (FRET); and a platform from LI-COR Biosciences using dye-quencher nucleotides in sequencing reactions.

Other NGS methods include, but are not limited to, nanopore sequencing, sequencing by hybridization, nanotransistor-array-based sequencing, Polony sequencing, scanning electron tunneling microscopy (STM)-based sequencing, and nanowire molecular sensor-based sequencing.

Nanopore sequencing involves electrophoresis of nucleic acid molecules in solution through nano-scale pores that provide a highly airtight area for analysis of single-nucleic-acid polymers. Representative nanopore sequencing methods are described in Branton D. et al., Nat. Biotechnol. 2008; 26(10):1146-53] and elsewhere.

Sequencing by hybridization is a non-enzymatic method using DNA microarrays. Typically, a single pool of DNA is fluorescently labeled and hybridized into an array containing a known sequence. The hybridization signal from a given spot on the array can be used to identify the DNA sequence. Binding of one strand of DNA to another strand complementary thereto in a DNA double strand is sensitive even to single-base mismatches when the hybrid region is short or when a specified mismatch detection protein is present. Representative hybridization sequencing methods are described, for example, in Hanna G. J. et al., J. Clin. Microbiol. 2000; 38(7): 2715-21; and Edwards J. R. et al., Mut. Res. 2005; 573(1-2): 3-12.

Polony sequencing is based on Polony amplification and multiple single-base-extension (FISSEQ). Polony amplification is a method of amplifying DNA *in situ* on a polyacrylamide film. Representative Polony sequencing methods are described, for example, in US Patent Application Publication No. 2007/0087362.

Nanotransistor-array-based devices such as carbon nanotube field effect transistors (CNTFETs) can also be used for NGS. For example, DNA molecules are extended and driven across nanotubes by microfabricated electrodes. DNA molecules sequentially contact the carbon nanotube surface, and a difference in current flow from the respective bases is created due to charge transfer between the DNA molecule and the nanotube. DNA is sequenced by recording the difference. Representative nanotransistor-array-based sequencing methods are described, for example, in US Patent Publication No. 2006/0246497.

Scanning tunneling microscopy (STM) can also be used for NGS. Using a piezoelectrically controlled probe that performs a raster scan of the specimen, STM forms an image on the surface thereof. STM can be used to image the physical properties of single DNA molecules, causing coherent electron tunneling imaging and spectroscopy, for example, by integrating a flexible actuator-driven gap with a scanning tunneling microscope. Representative sequencing methods using STM are described, for example, in US Patent Application Publication No. 2007/0194225.

Molecular analysis devices consisting of nanowire-molecular sensors can also be used for NGS. Such devices can detect the interaction of nitrogenous substances disposed on nucleic acid molecules and nanowires such as DNA. Molecular guides are disposed to guide molecules near the molecular sensors to allow interaction and subsequent detection. Representative sequencing methods using nanowire molecular sensors are described, for example, in US Patent Application Publication No. 2006/0275779.

Double-stranded sequencing may be used for NGS. Double-stranded sequencing uses blocking and unblocking primers to sequence both the sense and antisense strands of DNA. Typically, this method includes: annealing an unblocking primer to a first strand of a nucleic acid; annealing a second blocking primer to a second strand of the nucleic acid; extending the nucleic acid along the first strand with a polymerase; terminating the first sequencing primer; deblocking the second primer; and extending the nucleic acid along the second strand. Representative double-stranded sequencing methods are described, for example, in US Pat. No. 7,244,567.

Data analysis stage.

After NGS reads are formed, they are aligned or *de novo* assembled to a known reference sequence.

For example, identification of genetic modifications such as single-nucleotide polymorphisms and structural variants in a sample (e.g., a tumor sample) can be performed by aligning NGS reads to a reference sequence (e.g., a wild-type sequence). A method of aligning NGS reads to sequences is described, for example, in Trapnell C. and Salzberg S.L. Nature Biotech., 2009, 27:455-457.

Examples of de *novo* assembly are described, for example, in Warren R. et al., Bioinformatics, 2007, 23:500-501; Butler J. et al., Genome Res., 2008, 18:810-820; and Zerbino D.R. and Birney E., Genome Res., 2008, 18:821-829.

Sequence alignment or assembly can be performed using read data from one or more NGS platforms, for example, by mixing Roche/454 and Illumina/Solexa read data. In the present invention, the alignment may be performed using the BWA algorithm and the hg19 sequence, but is not limited thereto.

In the present invention, the sequence alignment of step b) includes a computational method or approach using a computer algorithm to determine the case where there is the possibility that a sequence (e.g., a short-read sequence obtained, for example, through next-generation sequencing) is derived from the genome or the case where there is identity therebetween by evaluating the similarity between a read sequence and a reference sequence. Various algorithms may be applied to the sequence alignment problem. Some algorithms are relatively slow, but enable relatively high specificity. These include, for example, dynamic-programming-based algorithms. Dynamic programming is a method of solving complicated problems by segmenting them into simpler steps. Other approaches are more efficient, but are typically not exhaustive, and include, for example, heuristic algorithms and probabilistic methods designed for massive database searches.

Typically, the alignment process may include two steps, namely candidate screening and sequence alignment. Candidate screening reduces the search space for sequence alignments from the entire genome in order to obtain a shorter list of possible alignment positions. As the term literally implies, sequence alignment includes aligning sequences including the sequences obtained during candidate screening. This may be performed using broad alignment (e.g., Needleman-Wunsch alignment) or local alignment (e.g., Smith-Waterman alignment).

Most attribute sorting algorithms may have one of three types based on the indexing method: algorithms based on hash tables (e.g. BLAST, ELAND, SOAP), suffix trees (e.g. Bowtie, BWA), and merge sort (for example, slider). Short read sequences are typically used for alignment. Examples of sequence alignment algorithms/programs for short-read sequences include, but are not limited to, BFAST (Homer N. et al., PLoS One. 2009;4(11):e7767), BLASTN (from blast.ncbi.nlm.nih.gov on the world wide web), BLAT (Kent W. J. Genome Res. 2002;12(4):656-64), Bowtie (Langmead B. et al., Genome Biol. 2009;10(3):R25), BWA (Li H. and Durbin R., Bioinformatics, 2009, 25:1754-60), BWA-SW (Li H. and Durbin R., Bioinformatics, 2010;26(5):589-95), CloudBurst (Schatz M. C., Bioinformatics, 2009;25(11):1363-9), Corona Lite (Applied Biosystems, Carlsbad, California, USA), CASHX (Fahlgren N. et al., RNA, 2009; 15, 992-1002), CUDA-EC (Shi H. et al., J. Comput. Biol. 2010;17(4):603-15), ELAND (bioit.dbi.udel.edu/howto/eland on the world wide web), GNUMAP (Clement N. L. et al., Bioinformatics. 2010;26(1):38-45), GMAP (Wu T.D. and Watanabe C.K., Bioinformatics, 2005;21(9):1859-75), GSNAP (Wu T.D. and Nacu S., Bioinformatics, 2010;26(7):873-81), Geneious Assembler (Biomatters Ltd., Oakland, New Zealand), LAST, MAQ (Li H. et al., Genome Res. 2008;18(11):1851-8), Mega-BLAST (at ncbi.nlm.nih.gov/blast/megablast.shtml on the world wide web), MOM (Eaves H.L. and Gao Y. Bioinformatics. 2009;25(7):969-70), MOSAIK (at bioinformatics.bc.edu/marthlab/Mosaik on the world wide web), NovoAlign (at novocraft.com/main/index.php on the world wide web), PALMapper (at fml.tuebingen.mpg.de/raetsch/suppl/palmapper on the world wide web), PASS (Campagna D. et al., Bioinformatics, 2009;25(7):967-8), PatMaN (Prufer K. et al., Bioinformatics, 2008; 24(13):1530-1), PerM (Chen Y. et al., Bioinformatics, 2009, 25 (19): 2514-2521), ProbeMatch (Kim Y. J. et al., Bioinformatics. 2009;25(11):1424-5), QPalma (de Bona F. et al., Bioinformatics, 2008, 24(16): i174), RazerS (Weese D. et al., Genome Research, 2009, 19:1646-1654), RMAP (Smith A.D. et al., Bioinformatics, 2009;25(21):2841-2), SeqMap (Jiang H. et al., Bioinformatics, 2008;24:2395-2396), Shrec (Salmela L., Bioinformatics, 2010;26(10):1284-90), SHRiMP (Rumble S.M. et al., PLoS Comput. Biol., 2009, 5(5):e1000386), SLIDER (Malhis N. et al., Bioinformatics, 2009, 25 (1): 6-13), SLIM Search (Muller T. et al., Bioinformatics, 2001;17 Suppl 1:S182-9), SOAP (Li R. et al., Bioinformatics, 2008;24(5):713-4), SOAP2 (Li R. et al., Bioinformatics, 2009;25(15):1966-7), SOCS (Ondov B.D. et al., Bioinformatics, 2008; 24(23):2776-7), SSAHA (Ning Z. et al., Genome Res. 2001;11(10):1725-9), SSAHA2 (Ning Z. et al., Genome Res. 2001;11(10):1725-9), Stampy (Lunter G. and Goodson M., Genome Res. 2010, epub ahead of print), Taipan (at taipan.sourceforge.net on the world wide web), UGENE (at ugene.unipro.ru on the world wide web), XpressAlign (at bcgsc.ca/platform/bioinfo/software/XpressAlign on the world wide web), and ZOOM (Bioinformatics Solutions Inc., Waterloo, Ontario, Canada).

A sequence alignment algorithm may be selected based on a number of factors including, for example, the sequencing technique, length of reads, number of reads, available computing resources, and sensitivity/scoring requirements. Different sequence alignment algorithms can achieve different levels of speed, alignment sensitivity, and alignment specificity. Alignment specificity refers to the percentage of target sequence residues that are correctly aligned with the predicted alignment, as typically shown in the submission. Alignment sensitivity also refers to the percentage of target sequence residues that are aligned, as shown in typically predicted alignments in the submission.

Alignment algorithms such as ELAND or SOAP can be used to align short reads (e.g., from Illumina/Solexa sequencers) to a reference genome when the speed is the first factor to be considered. Alignment algorithms such as BLAST or Mega-BLAST are used to determine similarity using shorter reads (e.g., Roche FLX) when specificity is considered the most important factor, although these methods are slower. Alignment algorithms such as MAQ or NovoAlign can be used for single- or paired-end data when the quality score is important and accuracy is thus essential (e.g. in fast massive SNP searches). Alignment algorithms such as Bowtie or BWA use the Burrows-Wheeler Transform (BWT) and thus require a relatively small memory footprint. Alignment algorithms such as BFAST, PerM, SHRiMP, SOCS, or ZOOM map color space reads and thus can be used along with the SOLiD platform from ABI. In some applications, results from two or more sorting algorithms may be combined.

In the present invention, the length of the sequence information (reads) in step b) is 5 to 5,000 bp, and the number of sequence information (reads) that are used may be 5,000 to 5 million, but the invention is not limited thereto.

In the present invention, as the vectorized data in step c), any vectorized data that can be generated based on aligned nucleic acid fragments may be used without limitation, but the vectorized data is preferably a grand canyon plot (GC plot) or a genomic castle wall plot (GCW plot), but is not limited thereto.

In the present invention, the vectorized data is preferably an image, but is not limited thereto. An image is basically composed of pixels. If an image composed of pixels is vectorized, it may be expressed as a monochromatic 2D vector (black and white), a three-channel 2D vector (RGB colors), or a four-channel 2D vector (CMYK colors) depending on the type of image.

The vectorized data of the present invention is not limited to image data, and, for example, may be input data of an artificial intelligence model using an n-channel 2D vector (multi-channel vector) created by stacking n black-and-white images.

In the present invention, the GCW plot means a graph created by alternately aligning a normal chromosome and a chromosome of interest to clearly indicate the difference in each bin between the normal chromosome and the chromosome of interest. For example, when the RepFD for each chromosome is imaged, the RepFDs are similar in all chromosomes in a normal sample, so no up-down pattern occurs in a GCW plot thereof. However, in a chromosome aneuploidy sample, the RepFD value is lower in the aneuploid chromosomes than in normal chromosomes, so a clear up-down pattern occurs in the GCW plot. In order to maximize the difference in the pattern, an artificial intelligence model is taught a repeated alternate alignment of (normal chromosome, aneuploid chromosome)$_n$ to thereby enable the same to distinguish the normal sample from the chromosomal aneuploidy sample.

In the present invention, the GC plot is a plot generated by taking a specific section (either a constant bin or a bin of a different size) on the X-axis, and taking numerical values that can be expressed in terms of nucleic acid fragments, such as the distance between nucleic acid fragments or count of the nucleic acid fragments, on the Y-axis.

In the present invention, the method may further include, prior to step c), separating nucleic acid fragments satisfying a mapping quality score from the aligned nucleic acid fragments.

In the present invention, the mapping quality score may vary depending on a desired criterion, but is preferably 15 to 70, more preferably 50 to 70, and most preferably 60.

In the present invention, the GC plot of step c) is characterized in that the vectorized data is generated by calculating a distribution of aligned nucleic acid fragments in each chromosome bin based on the count of nucleic acid fragments in each bin or the distance between the nucleic acid fragments.

Vectorization of the calculated count of nucleic acid fragments or the calculated distance between nucleic acid fragments in the present invention may be performed using any known method for vectorizing the calculated value without limitation.

In the present invention, calculating the distribution of the aligned sequence information in each chromosome bin based on the count of nucleic acid fragments may be performed using a process including the following steps:

i) dividing chromosomes into predetermined bins;

ii) determining the count of nucleic acid fragments that are aligned in each bin;

iii) dividing the determined count of nucleic acid fragments in each bin by the total number of nucleic acid fragments in the sample to conduct normalization; and

iv) generating a GC plot with the order of respective bins on the X-axis and the normalized value calculated in step iii) on the Y-axis.

In the present invention, calculating the distribution of the aligned sequence information in each chromosome bin based on the distance between nucleic acid fragments may be performed using a process including the following steps:

i) dividing chromosomes into predetermined bins;

ii) determining the distance between nucleic acid fragments (fragment distance, FD) aligned in each bin;

iii) determining a representative distance between fragments (RepFD) of each bin based on the fragment distance calculated for each bin;

iv) normalizing RepFD by dividing the representative distance between fragments (RepFD) calculated in step iii) by a representative total nucleic acid fragment distance; and

v) generating a GC plot with the order of respective bins on an X-axis and the normalized value calculated in step iv) on a Y-axis.

In the present invention, the representative fragment distance (RepFD) includes at least one selected from the group consisting of a sum, difference, product, mean, median, quantile, minimum, maximum, variance, standard deviation, median absolute deviation, coefficient of variation of FD and combinations thereof, but is not limited thereto.

In the present invention, the predetermined bin may be 1 kb to 3 Gb, but is not limited thereto.

In the present invention, the method may further include grouping the nucleic acid fragments. The grouping may be performed based on the adapter sequence of the aligned nucleic acid fragments. The distance between the nucleic acid fragments for the selected sequence information may be calculated separately for nucleic acid fragments aligned in a forward direction and nucleic acid fragments aligned in a reverse direction.

In the present invention, the FD is defined as the distance between the representative positions of the i$^{th}$ nucleic acid fragment and the representative positions of at least one nucleic acid fragment selected from the i+1$^{th}$ to the n$^{th}$ nucleic acid fragments, among the obtained n nucleic acid fragments.

In the present invention, the FD may be one or more values selected from the group consisting of the sum, difference, product, mean, log of product, log of sum, median, quantile, minimum, maximum, variance, standard deviation, median absolute deviation and coefficient of variance of the distance between the representative positions of the 1st nucleic acid fragment and the representative positions of at least one nucleic acid fragment selected from the group consisting of the 2nd to nth nucleic acid fragments, among the obtained n nucleic acid fragments, and/or one or more reciprocals thereof, values calculated in consideration of weights, and statistical values, but the present invention is not limited thereto.

As used herein, the expression "one or more values... and/or one or more reciprocals thereof" is intended to mean that one of the numerical values described above or a combination of two or more thereof may be used.

As used herein, the expression "representative positions of the nucleic acid fragment" may be a value obtained by adding an arbitrary value to the median of the nucleic acid fragments or subtracting the arbitrary value therefrom.

The FD for the obtained n nucleic acid fragments may be defined as follows.

$$FD = Dist(Ri \sim Rj) \quad (1 < i < j < n),$$

wherein the Dist function calculates one or more values selected from the group consisting of the sum, difference, product, mean, log of product, log of sum, median, quantile, minimum, maximum, variance, standard deviation, median absolute deviation, and coefficient of variance of the differences between the alignment position values of all nucleic acid fragments between the two nucleic acid fragments Ri and Rj, and/or one or more reciprocals thereof, values calculated in consideration of weights, and statistical values, but is not limited thereto.

That is, as used herein, the FD (fragment distance) refers to the distance between aligned nucleic acid fragments. Here, the number of cases where nucleic acid fragments are selected for distance calculation may be defined as follows. When a total of N nucleic acid fragments are present, the number of combinations of distances between nucleic acid fragments is

$$\sum_{k=1}^{k-1} k.$$

That is, when i is 1, i+1 is 2, and the FD may be defined as the distance between the 1st nucleic acid fragment and one or more nucleic acid fragments selected from the 2nd to nth nucleic acid fragments.

In the present invention, the FD may be obtained by calculating the distance between a specific position inside the ith nucleic acid fragment and a specific position inside at least one of the i+1th to nth nucleic acid fragments.

For example, if a nucleic acid fragment has a length of 50 bp and is aligned at position 4,183 on chromosome 1, the genetic position values that can be used to calculate the distance between this nucleic acid fragment and another nucleic acid fragment are 4,183 and 4,232 on chromosome 1.

If a nucleic acid fragment having a length of 50 bp adjacent to the nucleic acid fragment is aligned at position 4,232 of chromosome 1, the genetic position values that can be used to calculate the distance between this nucleic acid fragment and another nucleic acid fragment are 4,232 and 4,281 of chromosome 1, and the FD between the two nucleic acid fragments is 1 to 99.

If another adjacent 50 bp nucleic acid fragment is aligned at position 4123 of chromosome 1, the genetic position values that can be used to calculate to calculate the distance between this nucleic acid fragment and another nucleic acid fragment are 4,123 and 4,172 of chromosome 1, the FD between the two nucleic acid fragments is 61 to 159, and the FD between the nucleic acid fragment and the first exemplary nucleic acid fragment is 12 to 110, the FD may be one or more selected from the group consisting of the sum, difference, product, mean, log of product, log of sum, median, quantile, minimum, maximum, variance, standard deviation, median absolute deviation and coefficient of variance of one within the range between the two FD values, and/or one or more reciprocals thereof, values calculated in consideration of weights, and statistical values, but are not limited thereto, and is preferably the reciprocal of one within the range of the two FD values, but is not limited thereto.

Preferably, in the present invention, the FD may be a value obtained by adding an arbitrary value to the median of the nucleic acid fragment or subtracting the arbitrary value therefrom.

In the present invention, the median of FD means the most centrally located value when the calculated FDs are arranged in order of size. For example, when there are three values, namely 1, 2, and 100, 2, which is central, is the median. If there is an even number of FDs, the median is determined as the mean of the two middle values. For example, if there are FDs of 1, 10, 90, and 200, the median is 50, which is the mean of 10 and 90.

In the present invention, the arbitrary value can be set without limitation, as long as it can be used to indicate the position of the nucleic acid fragment, but is preferably 0 to 5 kbp or 0 to 300% of the length of the nucleic acid fragment, 0 to 3 kbp or 0 to 200% of the length of the nucleic acid fragment, or 0 to 1 kbp or 0 to 100% of the length of the nucleic acid fragment, more preferably 0 to 500 bp or 0 to 50% of the length of the nucleic acid fragment, but is not limited thereto.

In the present invention, in paired-end sequencing, the FD may be derived based on position values of forward and reverse reads.

For example, if, in a pair of 50-bp-long paired-end reads, the forward read is aligned at position 4183 of chromosome 1 and the reverse read is aligned at position 4349, both ends of this nucleic acid fragment are at positions 4183 and 4349, and representative positions that can be used to calculate the nucleic acid fragment distance are 4183 and 4349. At this time, if, in another paired-end read pair adjacent to the nucleic acid fragment, the forward read is aligned at position 4349 of chromosome 1 and the reverse read is aligned at position 4515, the position values of the nucleic acid fragment are 4349 and 4515. The distance between the two nucleic acid fragments may be 0 to 333, and most preferably may be 166, which is the distance corresponding to the median of the respective nucleic acid fragments.

In the present invention, when sequence information is obtained through paired-end sequencing, the method may further include excluding nucleic acid fragments having a mapping quality score below a reference value from the calculation process.

In the present invention, in single-end sequencing, the FD may be derived based on one type of position value among forward and reverse reads.

In the present invention, in the single-end sequencing, if a position value is derived based on sequence information aligned in the forward direction, an arbitrary value is added thereto, and if a position value is derived based on sequence information aligned in the reverse direction, an arbitrary value is subtracted. The arbitrary value may be set without limitation, as long as the FD clearly indicates the position of the nucleic acid fragment, but is preferably 0 to 5 kbp or 0 to 300% of the length of the nucleic acid fragment, 0 to 3 kbp or 0 to 200% of the length of the nucleic acid fragment, or 0 to 1 kbp or 0 to 100% of the length of the nucleic acid fragment, more preferably 0 to 500 bp or 0 to 50% of the length of the nucleic acid fragment, but is not limited thereto.

Nucleic acids to be analyzed in the present invention may be sequenced and expressed in units called "reads". The reads may be divided into single-end sequencing reads (SE) and paired-end sequencing reads (PE) depending on the sequencing method. An SE-type read is a read obtained by sequencing one of a 5' and 3' end of a nucleic acid molecule to a predetermined length in a random direction, and a PE-type read is a read obtained by sequencing both 5' and 3' ends of a nucleic acid molecule to a predetermined length. It is well known to those skilled in the art that due to this difference, one read is generated from one nucleic acid fragment when sequencing in the SE mode, whereas a pair of two reads is generated from one nucleic acid fragment in the PE mode.

The most ideal method to accurately calculate the distance between nucleic acid fragments includes sequencing nucleic acid molecules from the beginning to the end, aligning the reads, and using the median (center) of the position values of the aligned reads. However, the method faces technical restrictions due to limitations on sequencing technology and the high cost thereof. Therefore, sequencing is performed using a method such as SE or PE. In the PE mode, the start and end positions of the nucleic acid molecule can be recognized, so the exact position (median) of the nucleic acid fragment can be determined through the combination of these values. In the SE mode, only information on one end of the nucleic acid fragment can be used, so there is a limitation on accuracy of calculation of the position (median).

Also, when calculating the distance between nucleic acid molecules using the end information of all reads sequenced (aligned) in both forward and reverse directions, an inaccurate value may be obtained due to the factor of the sequencing direction.

Therefore, for technical reasons related to the sequencing method, the 5' end of the forward read has a small position value and the 3' end of the reverse read has a large position value, compared to the central position value of the nucleic acid molecule. When an arbitrary value (extended bp) is added to the forward read and subtracted from the reverse read, using this feature, a value close to the central position of the nucleic acid molecule can be estimated.

That is, the arbitrary value (extended bp) may vary depending on the sample that is used, and cell-free nucleic acids are known to have an average nucleic acid length of about 166 bp, and thus the arbitrary value (extended bp) thereof is set to about 80 bp. If the experiment is performed using fragmentation (e.g. sonication) equipment, about half of the target length set during the fragmentation process may be set as extended bp.

In the present invention, the representative FD (RepFD) may be at least one selected from the group consisting of a sum, difference, product, mean, median, quantile, minimum, maximum, variance, standard deviation, median absolute deviation and coefficient of variance of FD, and/or a reciprocal thereof. Preferably, the representative FD (RepFD) may be a median or mean of FDs or a reciprocal thereof, but is not limited thereto.

In the present invention, the GCW plot is characterized in that vectorized data is generated by calculating the distance between the aligned nucleic acid fragments or the amount thereof in each chromosome bin, and alternately aligning the fragment distance of the normal chromosome with the fragment distance of the chromosome for which presence of aneuploidy is to be determined.

In the present invention, "alternate alignment" means aligning ([calculated value of normal chromosome], [calculated value of chromosome for which presence of aneuploidy is to be determined])$_n$ along the x-axis. For example, in alternate alignment, upon determining whether or not trisomy 21 is present, alignment is made along the x-axis in the order of RepFD of chromosome 1, RepFD of chromosome 21, RepFD of chromosome 2, RepFD of chromosome 21, RepFD of chromosome 3, and RepFD of chromosome 21.

In the present invention, the GCW plot may be created using a method including the following steps when the distance between the nucleic acid fragments is used as the calculated value.

i) calculating the distance between the aligned nucleic acid fragments (fragment distance, FD) for each chromosome;

ii) determining a representative fragment distance (RepFD) of each bin based on the distance calculated in step i);

iii) standardizing the RepFD for each chromosome determined in step ii);

iv) selecting a portion of the chromosomes excluding the chromosomes for which presence of aneuploidy is to be determined as control chromosomes; and

v) generating a GCW plot with the control chromosomes and the chromosomes for which the presence of aneuploidy is to be determined sequentially and alternately aligned on the X axis and the standardized calculated values of the respective chromosomes on the Y axis.

In the present invention, the standardization includes:

1) setting a reference group including normal samples from which chromosomal aneuploidies are not detected;

2) calculating a mean (reference_mean) and standard deviation (reference_standard_deviation) of RepFDs for respective chromosomes observed in the reference group;

3) conducting Z standardization by applying the reference_mean and reference_standard_deviation calculated in step 2) to Equation 1 below;

Equation 1:

$$Z_{chr} = ((RepFD_{chr} - Reference\_Mean_{chr}) / Reference\_Standard\_Deviation_{chr}) + 5$$

wherein, in a sample with a large amount of sequencing (number of reads), RepFD may be lowered and thus $Z_{chr}$ may be negative. When $Z_{chr}$ is negative, a problem in which an up-down pattern is reversed into a down-up pattern occurs in the process of dividing $Z_{chr}$ for the chromosome of the reference group calculated in step 3) by $Z_{chr}$ for the chromosome for which presence of aneuploidy is to be determined. For this reason, 5 is finally added to make all values positive.

4) dividing $Z_{chr}$ for each chromosome of the reference group calculated in step 3) by $Z_{chr}$ for the chromosome for which the presence of aneuploidy is to be determined.

Here, in step 4), $Z_{chr}$ for each chromosome of the reference group calculated in step 3) is divided by $Z_{chr}$ for the chromosome for which the presence of aneuploidy is to be determined in order to correct the change in the y-scale of the GCW plot depending on the amount of sequencing of the sample. Through this process, a GCW plot with a constant y-scale can be generated, regardless of the amount of sequencing of the sample.

In the present invention, the representative FD (RepFD) includes at least one selected from the group consisting of a sum, difference, product, mean, median, quantile, minimum, maximum, variance, standard deviation, median absolute deviation, and coefficient of variance of FD and/or a reciprocal thereof, and is preferably a median or mean of FDs or a reciprocal thereof, but is not limited thereto.

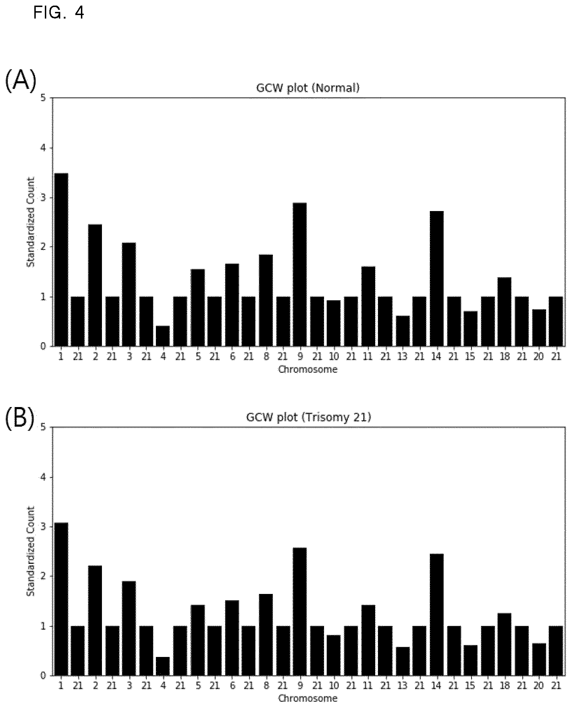

In the present invention, when the GCW plot is drawn based on the amount of nucleic acid fragments, there is a difference in height with respect to the bar of the control chromosome, so the artificial intelligence model can perform learning thereon (FIG. 4).

In the present invention, the control chromosome group may be any combination of chromosomes excluding the chromosome group to be detected, preferably chromosome 1, 2, 3, 4, 5, 6, 8, 9, 10, 11, 13, 14, 15, 18, or 20, but is not limited thereto.

In the present invention, the vectorized data may include a plurality of chromosome-specific plots in one image.

In the present invention, any artificial intelligence model may be used without limitation in step (d), as long as it is a model that can learn to distinguish between an image of normal chromosomes and an image of abnormal chromosomes, and is preferably a deep-learning model.

In the present invention, any artificial intelligence model may be used without limitation, as long as it is an artificial neural network algorithm capable of analyzing vectorized data based on an artificial neural network, and is preferably selected from the group consisting of a convolutional neural network (CNN), a deep neural network (DNN), a recurrent neural network (RNN), and an autoencoder, but is not limited thereto.

In the present invention, the recurrent neural network is selected from the group consisting of a longshort term memory (LSTM) neural network, a gated recurrent unit (GRU) neural network, a vanilla recurrent neural network, and an attentive recurrent neural network.

In the present invention, when the artificial intelligence model is CNN, the loss function for performing binary classification is represented by Equation 2 below, and the loss function for performing multi-class classification is represented by Equation 3 below.

Equation 2: Binary classification

$$\text{loss(model(x),y)} = -\frac{1}{n}\left[\sum_{i=1}^{n}\left(y_i \log\left(model(x_i)\right) + (1 - y_i)\log\left(1 - model(x_i)\right)\right)\right]$$

Model $(x_i)$ = Artificial intelligence model output in response to $i^{th}$ input

y = Actual label value

n = Number of input data

Equation 3: Multi-class classification

$$\text{loss(model(x),y)} = -\frac{1}{n}\sum_{i=1}^{n}\left(\sum_{j=1}^{c}\left(y_{ij} \log(model(x_i))_j\right)\right)$$

Model $(x_i)_j$ = $j^{th}$ artificial intelligence model output in response to $i^{th}$ input

y = Actual label value

n = Number of input data

c = Number of classes

In the present invention, the binary classification means that the artificial intelligence model learns to identify one chromosomal abnormality, and multi-class classification means that the artificial intelligence model learns to distinguish between two or more chromosomal abnormalities.

In the present invention, when the artificial intelligence model is a CNN, learning includes the following steps:

i) classifying the generated GC and GCW plots into training, validation, and test data,
wherein the training data is used when the CNN model is trained, the validation data is used for hyper-parameter tuning validation, and the test data is used for performance evaluation after optimal model production; and

ii) constructing an optimal CNN model through hyper-parameter tuning and training; and

iii) comparing the performance of multiple models obtained through hyper-parameter tuning using validation data and determining the model having the best validation data to be the optimal model.

In the present invention, the hyper-parameter tuning is a process of optimizing the values of various parameters (the number of convolution layers, the number of dense layers, the number of convolution filters, etc.) constituting the CNN model. The hyper-parameter tuning is performed using Bayesian optimization and grid search methods.

In the present invention, the internal parameters (weights) of the CNN model are optimized using predetermined hyper-parameters, and it is determined that the model is over-fit when validation loss starts to increase compared to training loss. Training is stopped prior to this determination.

In the present invention, any value resulting from analysis of the input vectorized data by the artificial intelligence model in step d) may be used without limitation, as long as it is a specific score or real number, and the value is preferably a deep probability index (DPI), but is not limited thereto.

In the present invention, "deep probability index" means a value expressed as a probability value by adjusting the output of artificial intelligence to a scale of 0 to 1 using, for the last layer of the artificial intelligence model, a sigmoid function in the case of binary classification and a softmax function in the case of multi-class classification.

In the present invention, the cut-off value of step d) is 0.5. When the value derived through analysis by the artificial intelligence model is 0.5 or more, it is determined that there is a chromosomal abnormality.

In the present invention, the artificial intelligence model is trained to output a value close to 1 if there is aneuploidy and to output a value close to 0 if there is no aneuploidy. Therefore, performance (training, validation, test accuracy) is measured based on a cut-off value of 0.5. In other words, if the output value is 0.5 or more, it is determined that there is aneuploidy, and if it is less than 0.5, it is determined that there is no aneuploidy.

Here, it will be apparent to those skilled in the art that the cut-off value of 0.5 may be arbitrarily changed. For example, in an attempt to reduce false positives, the cut-off value may be set to be higher than 0.5 as a stricter criterion for determining aneuploidy, and in an attempt to reduce false negatives, the cut-off value may be set to be lower than 0.5 as a weaker criterion for determining aneuploidy.

Most preferably, the cut-off value can be set by determining the probability of the DPI by applying unseen data (data containing a solution that is different from that learned during learning) using the trained artificial intelligence model.

In another aspect, the present invention is directed to a device for determining a chromosomal abnormality based on artificial intelligence, the device including:

a decoder configured to extract nucleic acids from a biological sample and decode sequence information;

an aligner configured to align the decoded sequence with a reference genome database;

a data generator configured to generate vectorized data based on aligned nucleic acid fragments; and

a chromosomal abnormality determiner configured to input the generated vectorized data into a trained artificial intelligence model, analyze the data, and compare the resulting value with a cut-off value thereby to determine whether or not there is a chromosomal abnormality.

In another aspect, the present invention is directed to a computer-readable storage medium including an instruction configured to be executed by a processor for detecting a chromosomal abnormality through the following steps, including:

a) obtaining sequence information using extracted nucleic acids from a biological sample;

b) aligning the obtained sequence information (reads) with a reference genome database;

c) generating vectorized data based on the aligned nucleic acid fragments; and

d) inputting the generated vectorized data into a trained artificial intelligence model, analyzing the data, and comparing the resulting value with a cut-off value to determine whether or not there is a chromosomal abnormality.

Example

Hereinafter, the present invention will be described in more detail with reference to examples. However, it will be obvious to those skilled in the art that these examples are provided only for illustration of the present invention, and should not be construed as limiting the scope of the present invention.

Example 1. Extracting DNA from blood to perform next-generation sequencing

10 mL of blood was collected from each of 7,931 normal subjects, 170 Trisomy 21 patients, 58 Trisomy 18 patients, and 16 Trisomy 13 patients, and stored in an EDTA tube. Within 2 hours after blood collection, only the plasma was primarily centrifuged at 1,200g and 4°C for 15 minutes, and then the primarily centrifuged plasma was secondarily centrifuged at 16,000g and 4°C for 10 minutes to isolate the plasma supernatant excluding the precipitate. Cell-free DNA was extracted from the isolated plasma using a Tiangenmicro DNA kit (Tiangen), a library preparation process was performed using a TruSeq Nano DNA HT library prep kit (Illumina), and then single-end (SE) data was generated using a Nextseq 500 device (Illumina). As a result, about 13 million reads were produced for each sample.

Example 2. Construction of trisomy 21 deep-learning model using GC plot based on nucleic acid fragment distance and performance testing

The performance of the DPI value was tested using normal samples (n=961) and T21 samples (n=170). All samples were divided into training, validation, and test groups. The models were constructed using the training samples, and then the performance of the models constructed using the training samples was evaluated using the samples of the validation and test groups.

[Table 1]

|  | Normal | T21 | Total | Baseline accuracy |
|---|---|---|---|---|
| Train | 589 | 93 | 682 | 86.4% |
| Validation | 156 | 34 | 190 | 82.1% |
| Test | 216 | 43 | 259 | 83.4% |
| Total | 961 | 170 | 1131 |  |

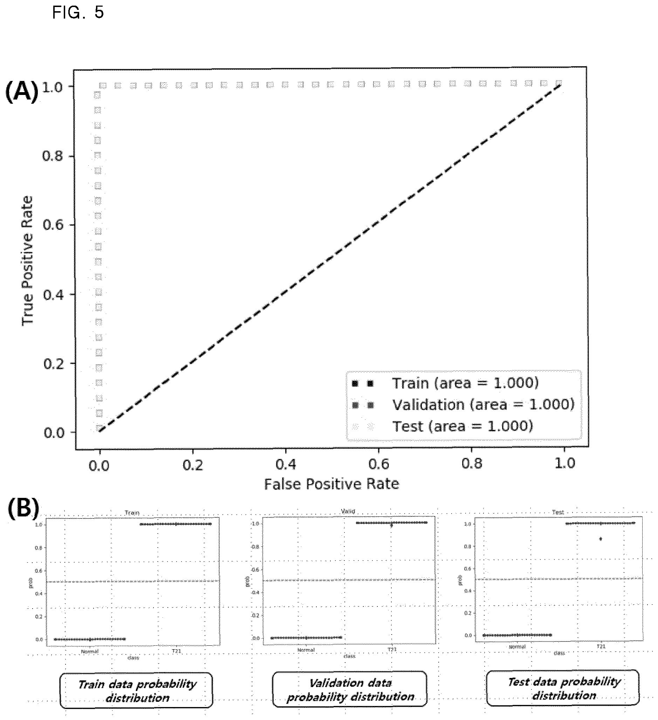

As a result, as can be seen from Table 2 and FIG. 5, accuracy was found to be 100%, 100%, and 100% in the training, validation, and test groups, respectively, and the AUC value, which indicates the result of ROC analysis, was found to be 1.0, 1.0, and 1.0 in the training, validation, and test groups, respectively.

[Table 2]

|  | Accuracy | AUC |
|---|---|---|
| Train | 100% | 1.0 |
| Validation | 100% | 1.0 |
| Test | 100% | 1.0 |

FIG. 5(A) shows the result of analysis using a receiver operating characteristic (ROC) curve to measure accuracy. It is determined that accuracy increases as the area under the curve (AUC) increases. The AUC has a value between 0 and 1, and when the label value is randomly predicted, the expected (baseline) AUC is 0.5, whereas when the label value is correctly predicted, the expected (baseline) AUC is 1.

FIG. 5(B) is a boxplot showing the probability value (DPI value) of chromosomal aneuploidy calculated in the artificial intelligence model of the present invention with respect to normal sample and trisomy 21 sample groups, wherein the red line represents the DPI cutoff of 0.5.

Example 3. Construction of trisomy 18 deep-learning model using GC plot based on nucleic acid fragment distance and performance testing

The performance of the DPI value was tested using normal samples (n=961) and T18 samples (n=58). All samples were divided into training, validation, and test groups. The models were constructed using the training samples, and then the performance of the models constructed using the training samples was evaluated using the samples of the validation and test groups.

[Table 3]

|  | Normal | T18 | Total | Baseline accuracy |
|---|---|---|---|---|
| Train | 589 | 28 | 617 | 95.5% |
| Validation | 156 | 18 | 174 | 89.7% |
| Test | 216 | 12 | 228 | 94.7% |
| Total | 961 | 58 | 1019 | |

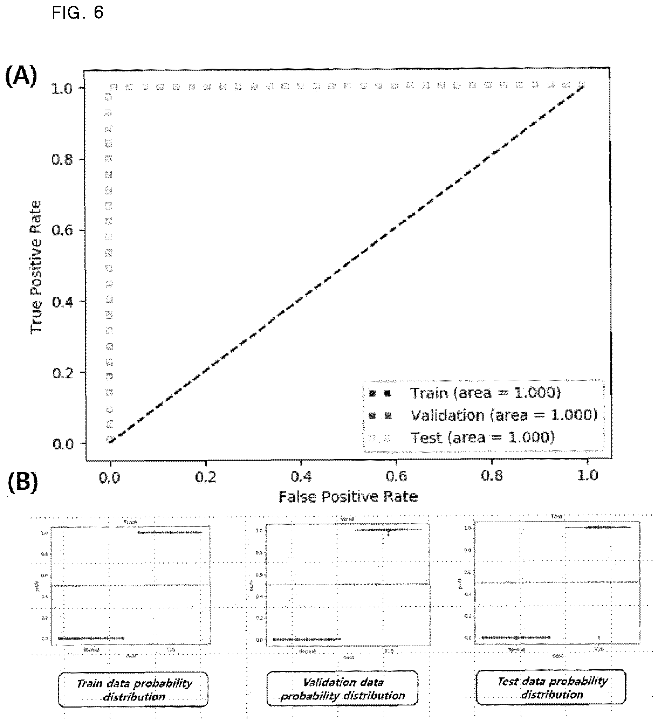

As a result, as can be seen from Table 4 and FIG. 6, accuracy was found to be 100%, 100%, and 100% in the training, validation, and test groups, respectively, and the AUC value, which indicates the result of ROC analysis, was found to be 1.0, 1.0, and 1.0 in the training, validation, and test groups, respectively.

[Table 4]

|  | Accuracy | AUC |
|---|---|---|
| Train | 100% | 1.0 |
| Validation | 100% | 1.0 |
| Test | 99.6% | 1.0 |

Example 4. Construction of trisomy 13 deep-learning model using GC plot based on nucleic acid fragment distance and performance testing

The performance of the DPI value was tested using normal samples (n=961) and T13 samples (n=16). All samples were divided into training, validation, and test groups. The models were constructed using the training samples, and then the performance of the models constructed using the training samples was evaluated using the samples of the validation and test groups.

[Table 5]

|  | Normal | T13 | Total | Baseline accuracy |
|---|---|---|---|---|
| Train | 589 | 7 | 596 | 98.8% |
| Validation | 156 | 5 | 161 | 96.9% |
| Test | 216 | 4 | 220 | 98.2% |
| Total | 961 | 16 | 977 | |

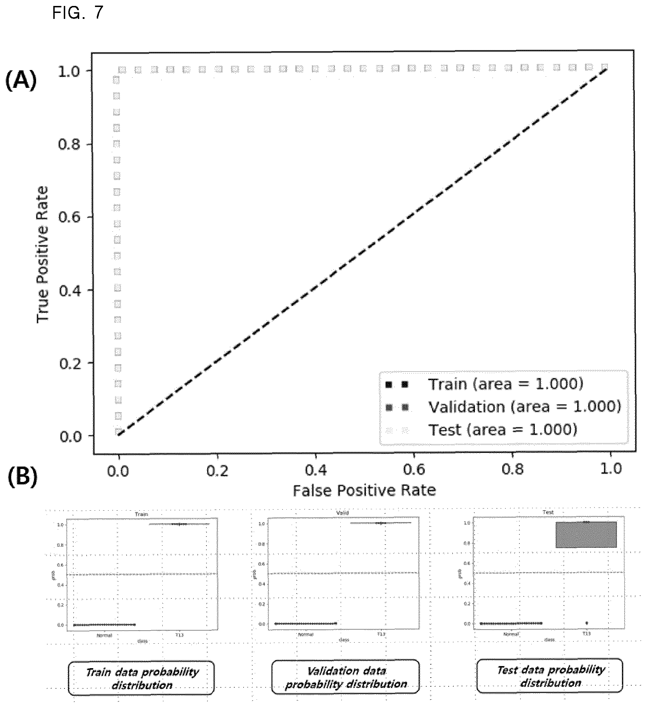

As a result, as can be seen from Table 6 and FIG. 7, accuracy was found to be 100%, 100%, and 100% in the training, validation, and test groups, respectively, and the AUC value, which indicates the result of ROC analysis, was found to be 1.0, 1.0, and 1.0 in the training, validation, and test groups, respectively.

[Table 6]

|  | Accuracy | AUC |
|---|---|---|
| Train | 100% | 1.0 |
| Validation | 100% | 1.0 |
| Test | 99.5% | 1.0 |

Example 5. Construction of trisomy 21 deep-learning model using GCW plot based on nucleic acid fragment distance and performance testing

Normal samples (n=716) were set as a reference group, a Z-score was calculated, and a GCW plot was generated using the Z-score. The nucleic acid fragment distance for calculating the Z score used herein was the median of multiple distances between nucleic acid fragments. The performance of DPI values was tested using normal samples (n=7,215) and T21 samples (n=168) not included in the reference group for Z score calculation. All samples were divided into training, validation, and test groups. The models were constructed using the training samples, and then the performance of the models constructed using the training samples was evaluated using the samples of the validation and test groups.

[Table 7]

|  | Normal | T21 | Total | Baseline accuracy |
|---|---|---|---|---|
| Train | 4,362 | 92 | 4,454 | 97.9% |
| Validation | 1,110 | 34 | 1,144 | 97.0% |
| Test | 1,743 | 42 | 1,785 | 97.7% |
| Total | 7,215 | 168 | 7,383 | 97.7% |

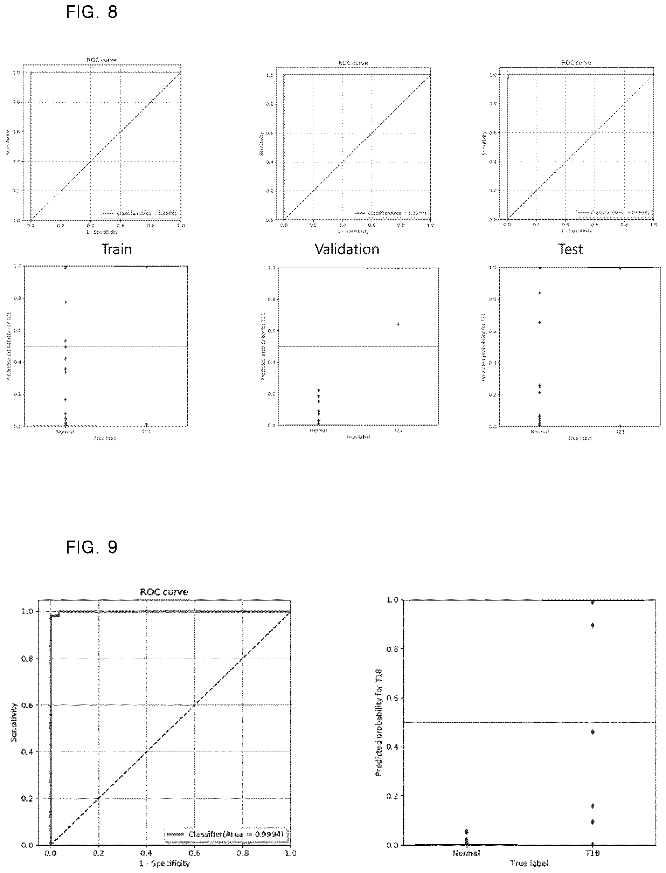

As a result, as can be seen from Table 8 and FIG. 8, accuracy was found to be 99.8%, 100%, and 99.8% in the training, validation, and test groups, respectively, and the AUC value, which indicates the result of ROC analysis, was found to be 0.9999, 1.0, and 0.9998 in the training, validation, and test groups, respectively.

[Table 8]

|  | Accuracy | AUC |
|---|---|---|
| Train | 99.8% | 0.9999 |
| Validation | 100% | 1.0 |
| Test | 99.8% | 0.9998 |

Example 6. Construction of trisomy 18 deep-learning model using GCW plot based on nucleic acid fragment distance and performance testing

Normal samples (n=716) were set as a reference group, a Z-score was calculated, and a GCW plot was generated using the Z-score. The nucleic acid fragment distance for calculating the Z score used herein was the median of multiple distances between nucleic acid fragments. The aneuploidy of chromosome 18 was predicted using the deep-learning model constructed in Example 5. The performance of DPI values was tested using normal samples (n=1,743) and T18 samples (n=58) not included in the reference group for Z score calculation.

[Table 9]

|  | Normal | T18 | Total | Baseline accuracy |
|---|---|---|---|---|
| Test | 1,743 | 58 | 1,801 | 96.7% |
| Total | 1,743 | 58 | 1,801 | 96.7% |

As a result, as can be seen from Table 10 and FIG. 9, accuracy was found to be 99.8% in the test group, and the AUC value, which indicates the result of ROC analysis, was found to be 0.9994.

[Table 10]

|  | Accuracy | AUC |
|---|---|---|
| Test | 99.8% | 0.9994 |

Example 7. Construction of trisomy 13 deep-learning model using GCW plot based on nucleic acid fragment distance and performance testing

Normal samples (n=716) were set as a reference group, a Z-score was calculated, and a GCW plot was generated using the Z-score. The nucleic acid fragment distance for calculating the Z score used herein was the median of multiple distances between nucleic acid fragments. The aneuploidy of chromosome 13 was predicted using the deep-learning model constructed in Example 5. The performance of DPI values was tested using normal samples (n=1,743) and T13 samples (n=16) not included in the reference group for Z score calculation.

[Table 11]

|  | Normal | T13 | Total | Baseline accuracy |
|---|---|---|---|---|
| Test | 1,743 | 16 | 1,759 | 99.9% |
| Total | 1,743 | 16 | 1,759 | 99.9% |

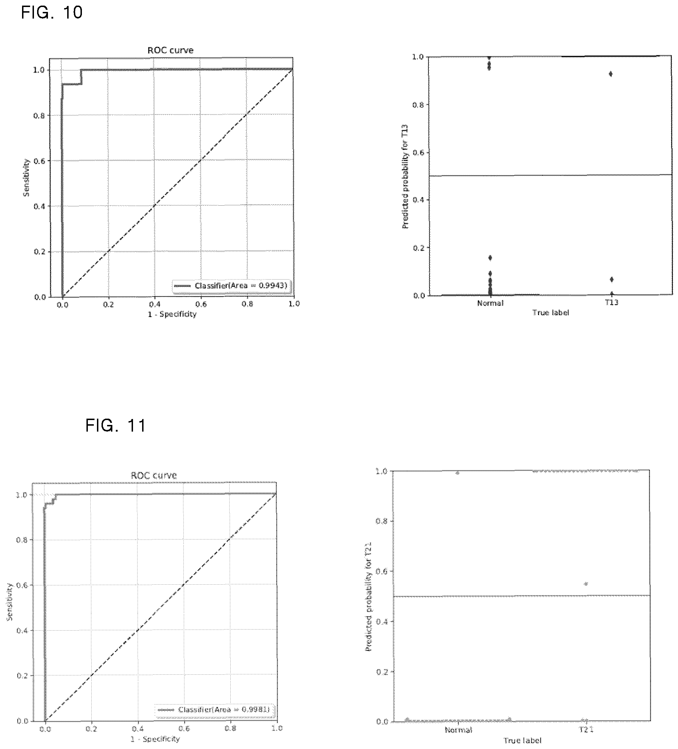

As a result, as can be seen from Table 12 and FIG. 10, accuracy was found to be 99.7% in the test group, and the AUC value, which indicates the result of ROC analysis, was found to be 0.9943.

[Table 12]

|  | Accuracy | AUC |
|---|---|---|
| Test | 99.7% | 0.9943 |

Example 8. Evaluation of downsampling performance using GCW plot based on nucleic acid fragment distance

The read coverage was reduced by half (from about 10,000,000 reads or more to 5,000,000 reads), and a GCW plot was drawn to detect chromosomal aneuploidy. The result showed that performance was maintained at a level similar to that before the reduction of read coverage. Trisomy 21 detection performance when the coverage is reduced is shown in Table 13 below.

[Table 13]

|  | Normal | T21 | Total | Baseline accuracy |
|---|---|---|---|---|
| Test | 158 | 49 | 207 | 76.3% |
| Total | 158 | 49 | 207 | 76.3% |

As a result, accuracy was found to be 98.6% in the test group, and the AUC value, which indicates the result of ROC analysis, was found to be 0.998 (Table 14 and FIG. 11).

[Table 14]

|  | Accuracy | AUC |
|---|---|---|
| Test | 98.6% | 0.9981 |

Example 9. Construction of trisomy 21 deep-learning model using GCW plot based on reciprocal value of nucleic acid fragment distance and performance testing

Normal samples (n=700) were set as a reference group, a Z-score was calculated, and a GCW plot was generated using the Z-score. The nucleic acid fragment distance for calculating the Z score used herein was the median of multiple distances between nucleic acid fragments. The performance of DPI values was tested using normal samples (n=991) and T21 samples (n=163) not included in the reference group for Z score calculation. All samples were divided into training, validation, and test groups. The models were constructed using the training samples, and then the performance of the models constructed using the training samples was evaluated using the samples of the validation and test groups.

[Table 15]

|  | Normal | T21 | Total | Baseline accuracy |
|---|---|---|---|---|
| Train | 485 | 79 | 564 | 84.5% |
| Validation | 208 | 35 | 243 | 85.6% |
| Test | 298 | 49 | 347 | 85.9% |
| Total | 991 | 163 | 1154 | 85.9% |

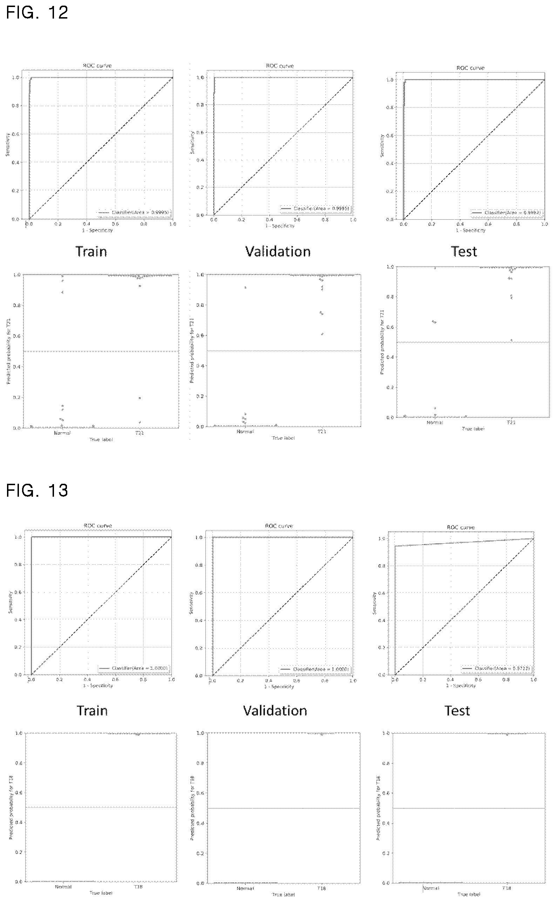

As a result, as can be seen from Table 16 and FIG. 12, accuracy was found to be 99.1%, 99.5%, and 99.1% in the training, validation, and test groups, respectively, and the AUC value, which indicates the result of ROC analysis, was found to be 0.9999, 0.999, and 0.999 in the training, validation, and test groups, respectively.

[Table 16]

|  | Accuracy | AUC |
|---|---|---|
| **Train** | 99.1% | 0.999 |
| **Validation** | 99.5% | 0.999 |
| **Test** | 99.1% | 0.999 |

Example 10. Construction of trisomy 18 deep-learning model using GCW plot based on reciprocal value of nucleic acid fragment distance and performance testing

Normal samples (n=700) were set as a reference group, a Z-score was calculated, and a GCW plot was generated using the Z-score. The nucleic acid fragment distance for calculating the Z score used herein was the median of multiple distances between nucleic acid fragments. The performance of DPI values was tested using normal samples (n=991) and T18 samples (n=57) not included in the reference group for Z score calculation. All samples were divided into training, validation, and test groups. The models were constructed using the training samples, and then the performance of the models constructed using the training samples was evaluated using the samples of the validation and test groups.

[Table 17]

|  | Normal | T18 | Total | Baseline accuracy |
|---|---|---|---|---|
| **Train** | 485 | 27 | 512 | 94.7% |
| **Validation** | 208 | 12 | 220 | 94.5% |
| **Test** | 298 | 18 | 316 | 94.3% |
| **Total** | 991 | 57 | 1048 | 94.6% |

As a result, as can be seen from Table 17 and FIG. 13, accuracy was found to be 100%, 100%, and 99.6% in the training, validation, and test groups, respectively, and the AUC value, which indicates the result of ROC analysis, was found to be 1.0, 1.0, and 0.972 in the training, validation, and test groups, respectively.

[Table 18]

|  | Accuracy | AUC |
|---|---|---|
| **Train** | 100% | 1 |
| **Validation** | 100% | 1 |
| **Test** | 99.6% | 0.972 |

Example 11. Construction of trisomy 13 deep-learning model using GCW plot based on reciprocal value of nucleic acid fragment distance and performance testing

Normal samples (n=700) were set as a reference group, a Z-score was calculated, and a GCW plot was generated using the Z-score. The nucleic acid fragment distance for calculating the Z score used herein was the median of multiple distances between nucleic acid fragments. The performance of DPI values was tested using normal samples (n=991) and T13 samples (n=16) not included in the reference group for Z score calculation. All samples were divided into training, validation, and test groups. The models were constructed using the training samples, and then the performance of the models constructed using the training samples was evaluated using the samples of the validation and test groups.

[Table 19]

|  | Normal | T13 | Total | Baseline accuracy |
|---|---|---|---|---|
| **Train** | 485 | 7 | 492 | 98.6% |
| **Validation** | 208 | 4 | 212 | 98.1 % |
| **Test** | 298 | 5 | 303 | 98.3% |
| **Total** | 991 | 16 | 1007 | 98.4% |

As a result, as can be seen from Table 20 and FIG. 13, accuracy was found to be 98.7%, 98.5%, and 99.0% in the training, validation, and test groups, respectively, and the AUC value, which indicates the result of ROC analysis, was found to be 0.996, 0.995, and 1.0 in the training, validation, and test groups, respectively.

[Table 20]

|  | Accuracy | AUC |
|---|---|---|
| **Train** | 98.7% | 0.996 |
| **Validation** | 98.5% | 0.995 |
| **Test** | 99.0% | 1.0 |

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

[Industrial applicability]

The method of determining a chromosomal abnormality based on artificial intelligence according to the present invention includes generating vectorized data and analyzing the same using an AI algorithm, and thus is useful, because it can convey a consistent effect even in the case of low read coverage, compared to when readrelated values are used as standardized values one by one in the conventional detection method that determines the amount of chromosomes based on the read count or the distance between aligned reads.

**Claims**

**1.** A method of detecting a chromosomal abnormality based on artificial intelligence, the method comprising:

a) obtaining sequence information using extracted nucleic acids from a biological sample;
b) aligning the obtained sequence information (reads) with a reference genome database;
c) generating vectorized data using nucleic acid fragments based on the aligned sequence information (reads); and
d) inputting the generated vectorized data into a trained artificial intelligence model, analyzing the data, and comparing the resulting value with a cut-off value to determine whether or not a chromosomal abnormality is present.

**2.** The method according to claim 1, wherein step (a) comprises:

(a-i) obtaining nucleic acids from blood, semen, vaginal cells, hair, saliva, urine, oral cells, amniotic fluid containing placental cells or fetal cells, tissue cells, or a mixture thereof;
(a-ii) obtaining purified nucleic acids by removing proteins, fats, and other residues from the obtained nucleic acids using a salting-out method, a column chromatography method, or a bead method;
(a-iii) preparing a single-end sequencing or paired-end sequencing library for the purified nucleic acids or nucleic acids randomly fragmented by an enzymatic digestion, pulverization, or hydroshear method;
(a-iv) reacting the prepared library with a next-generation sequencer; and
(a-v) obtaining sequence information (reads) of the nucleic acids in the next-generation sequencer.

3. The method according to claim 1, wherein the vectorized data of step (c) is a grand canyon plot (GC plot) or a genomic castle wall plot (GCW plot).

4. The method according to claim 3, wherein the GC plot is **characterized in that** the vectorized data is generated by calculating a distribution of aligned nucleic acid fragments in each chromosome bin based on the count of nucleic acid fragments in each bin or the distance between the nucleic acid fragments.

5. The method according to claim 4, wherein the calculating the distribution of the aligned sequence information in each chromosome bin based on the count of nucleic acid fragments is performed using a process including the following steps:

    i) dividing chromosomes into predetermined bins;
    ii) determining the count of nucleic acid fragments aligned in each bin;
    iii) dividing the determined count of nucleic acid fragments in each bin by a total number of nucleic acid fragments in the sample to conduct normalization; and
    iv) creating a GC plot with an order of respective bins on an X-axis and a normalized value calculated in step iii) on a Y-axis.

6. The method according to claim 4, wherein the calculating the distribution of the aligned sequence information in each chromosome bin based on the distance between nucleic acid fragments is performed using a process including the following steps:

    i) dividing chromosomes into predetermined bins;
    ii) calculating the distance between nucleic acid fragments (fragment distance, FD) aligned in each bin;
    iii) determining a representative distance between fragments (RepFD) of each bin based on the fragment distance calculated in each bin;
    iv) normalizing RepFD by dividing the representative distance between fragments (RepFD) calculated in step iii) by a representative total nucleic acid fragment distance; and
    v) generating a GC plot with the order of respective bins on an X-axis and the normalized value calculated in step iv) on a Y-axis.

7. The method according to claim 6, wherein the representative FD (RepFD) comprises at least one selected from the group consisting of a sum, difference, product, mean, median, quantile, minimum, maximum, variance, standard deviation, median absolute deviation, coefficient of variance of FD, a reciprocal thereof and a combination thereof.

8. The method according to claim 3, wherein the GCW plot is a graph created by calculating a distance between the aligned nucleic acid fragments in each chromosome and alternately aligning a fragment distance of a normal chromosome with a fragment distance of a chromosome for which presence of aneuploidy is to be determined.

9. The method according to claim 8, wherein the GCW plot is created using a process including the following steps:

    i) calculating the distance between the aligned nucleic acid fragments for each chromosome;
    ii) determining a representative distance between fragments for each bin based on the distance calculated in step i);
    iii) standardizing the representative distance between fragments in each chromosome determined in step ii) ;
    iv) selecting a portion of chromosomes excluding chromosomes for which presence of aneuploidy is to be determined as control chromosomes; and
    v) generating a GCW plot with the control chromosomes and the chromosomes for which presence of aneuploidy is to be determined sequentially and alternately aligned on the X axis and the standardized calculated values of the respective chromosomes on the Y axis.

10. The method according to claim 9, wherein the standardizing the representative distance between fragments of step iii) comprises:

    1) setting a reference group including normal samples from which chromosomal aneuploidies are not detected;
    2) calculating a mean (reference_mean) and standard deviation (reference_standard_deviation) of RepFDs for respective chromosomes observed in the reference group;
    3) conducting Z standardization by applying the reference_mean and reference_standard_deviation calculated in step 2) to Equation 1 below; and

```
Equation 1:


Z_chr        =        ((RepFD_chr        -        Reference_Mean_chr)

/Reference_Standard_Deviation_chr) + 5
```

4) dividing $Z_{chr}$ for the chromosome of the reference group calculated in step 3) by $Z_{chr}$ for the chromosome for which presence of aneuploidy is to be determined.

11. The method according to claim 9, wherein the representative FD (RepFD) comprises at least one selected from the group consisting of a sum, difference, product, mean, median, quantile, minimum, maximum, variance, standard deviation, median absolute deviation and coefficient of variance of the fragment distance (FD), a reciprocal thereof, and a combination thereof.

12. The method according to claim 1, wherein the artificial intelligence model of step (d) is trained to distinguish between vectorized data of normal chromosomes and vectorized data of abnormal chromosomes.

13. The method according to claim 12, wherein the artificial intelligence model is selected from the group consisting of a convolutional neural network (CNN), a deep neural network (DNN), a recurrent neural network (RNN), and an autoencoder.

14. The method according to claim 12, wherein, when the artificial intelligence model is CNN and learns binary classification, loss function is represented by Equation 2 below, and when the artificial intelligence model is CNN and learns multi-class classification, loss function is represented by Equation 3 below.

```
Equation 2: Binary classification
```

$$\text{loss}(\text{model}(x), y) = -\frac{1}{n}\left[\sum_{i=1}^{n}(y_i \log(model(x_i)) + (1 - y_i)\log(1 - model(x_i)))\right]$$

Model $(x_i)$ = Artificial intelligence model output in response to $i^{th}$ input
y = Actual label value
n = Number of input data

```
Equation 3: Multi-class classification
```

$$\text{loss}(\text{model}(x), y) = -\frac{1}{n}\sum_{i=1}^{n}\left(\sum_{j=1}^{c}(y_{ij}\log(model(x_i))_j)\right)$$

Model $(x_i)_j$ = $j^{th}$ artificial intelligence model output in response to $i^{th}$ input
y = Actual label value
n = Number of input data
c = Number of classes

15. The method according to claim 1, wherein the resulting value output through analysis of input vectorized data by the artificial intelligence model in step d) is a deep probability index (DPI).

16. The method according to claim 1, wherein the cut-off value of step d) is 0.5, and when the resulting value is 0.5 or more, it is determined that there is a chromosomal abnormality.

**17.** A device for determining a chromosomal abnormality based on artificial intelligence, the device comprising:

a decoder configured to extract nucleic acids from a biological sample and decode sequence information;
an aligner configured to align the decoded sequence with a reference genome database;
a data generator configured to generate vectorized data using nucleic acid fragments based on aligned sequence information (reads); and
a chromosomal abnormality determiner configured to input the generated vectorized data into a trained artificial intelligence model, analyze the data, and compare the resulting value with a cut-off value thereby to determine whether or not a chromosomal abnormality is present.

**18.** A computer-readable storage medium including an instruction configured to be executed by a processor for detecting a chromosomal abnormality through the following steps comprising:

a) obtaining sequence information using extracted nucleic acids from a biological sample;
b) aligning the obtained sequence information (reads) with a reference genome database;
c) generating vectorized data using nucleic acid fragments based on the aligned sequence information (reads); and
d) inputting the generated vectorized data into a trained artificial intelligence model, analyzing the data, and comparing the resulting value with a cut-off value to determine whether or not a chromosomal abnormality is present.

FIG. 1

```
┌─────────────────────────────────────┐
│   Extracting cell-free nucleic acid  │
│            from plasma               │
└─────────────────────────────────────┘
                  │
                  ▼
┌─────────────────────────────────────┐
│  Obtaining nucleic acid fragments by │
│     massive parallel sequencing      │
└─────────────────────────────────────┘
                  │
                  ▼
┌─────────────────────────────────────┐
│   Aligning nucleic acid fragment with│
│    human reference genome database   │
└─────────────────────────────────────┘
                  │
                  ▼
┌─────────────────────────────────────┐
│    Determining quality of aligned data│
└─────────────────────────────────────┘
                  │
                  ▼
┌─────────────────────────────────────┐
│ Data vectorization (i.e. GC plot, GCW plot)│
└─────────────────────────────────────┘
                  │
                  ▼
┌─────────────────────────────────────┐
│    CNN (convolutional neural network)│
└─────────────────────────────────────┘
                  │
                  ▼
┌─────────────────────────────────────┐
│ Calculation of deep probability (sigmoid)│
│            index (DPI)               │
└─────────────────────────────────────┘
                  │
                  ▼
┌─────────────────────────────────────┐
│        Determining whether           │
│       aneuploidy is present          │
└─────────────────────────────────────┘
```

FIG. 2

FIG. 3

(A)

(B)

FIG. 4

(A)

GCW plot (Normal)

(B)

GCW plot (Trisomy 21)

FIG. 5

(A)

(B)

Train data probability distribution

Validation data probability distribution

Test data probability distribution

FIG. 6

**(A)**

**(B)**

Train data probability distribution

Validation data probability distribution

Test data probability distribution

FIG. 7

**(A)**

**(B)**

Train data probability distribution

Validation data probability distribution

Test data probability distribution

FIG. 8

Train

Validation

Test

FIG. 9

FIG. 10

ROC curve

FIG. 11

ROC curve

FIG. 12

Train          Validation          Test

FIG. 13

Train          Validation          Test

FIG. 14

Train  Validation  Test

## INTERNATIONAL SEARCH REPORT

| International application No. |
|---|
| **PCT/KR2020/017065** |

| A. | CLASSIFICATION OF SUBJECT MATTER |
|---|---|

**G16B 20/20**(2019.01)i; **G16B 20/30**(2019.01)i; **G16B 30/10**(2019.01)i; **G16B 10/00**(2019.01)i; **G16B 40/20**(2019.01)i; **G06N 3/08**(2006.01)i

According to International Patent Classification (IPC) or to both national classification and IPC

| B. | FIELDS SEARCHED |
|---|---|

Minimum documentation searched (classification system followed by classification symbols)

G16B 20/20(2019.01); G06F 19/18(2011.01); G06F 19/22(2011.01); G06T 7/00(2006.01); G16C 10/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 염색체 이상(chromosomal abnormality), 리드(reads), 정렬(alignment), 벡터화된 데이터(vectorized data), 인공지능(artificial intelligence), 기준값(cut-off value)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-1686146 B1 (GREEN CROSS GENOME CORPORATION) 13 December 2016 (2016-12-13)<br>See abstract; and claims 1-16. | 1-18 |
| A | KR 10-2019-0049537 A (SEOUL NATIONAL UNIVERSITY R&DB FOUNDATION) 09 May 2019 (2019-05-09)<br>See entire document. | 1-18 |
| A | KR 10-2017-0092815 A (INDUSTRY-ACADEMIC COOPERATION FOUNDATION, YONSEI UNIVERSITY) 14 August 2017 (2017-08-14)<br>See entire document. | 1-18 |
| A | KR 10-2017-0017384 A (EONE DIAGNOMICS GENOME CENTER CO., LTD.) 15 February 2017 (2017-02-15)<br>See entire document. | 1-18 |
| A | KR 10-2015-0070111 A (PREMAITHA HEALTH LTD) 24 June 2015 (2015-06-24)<br>See entire document. | 1-18 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **12 March 2021** | **12 March 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2020/017065** |

**C. DOCUMENTS CONSIDERED TO BE RELEVANT**

| Category* | Citation of document, with indication, where appropriate, of the relevant passages | Relevant to claim No. |
| --- | --- | --- |
| A | WO 2016-139545 A1 (INDIAN INSTITUTE OF SCIENCE) 09 September 2016 (2016-09-09)<br>See entire document. | 1-18 |
| A | US 2014-0206006 A1 (XU, X. et al.) 24 July 2014 (2014-07-24)<br>See entire document. | 1-18 |

Form PCT/ISA/210 (second sheet) (July 2019)

**INTERNATIONAL SEARCH REPORT**
Information on patent family members

International application No.

**PCT/KR2020/017065**

| Patent document cited in search report | | | Publication date (day/month/year) | Patent family member(s) | | | Publication date (day/month/year) |
|---|---|---|---|---|---|---|---|
| KR | 10-1686146 | B1 | 13 December 2016 | None | | | |
| KR | 10-2019-0049537 | A | 09 May 2019 | None | | | |
| KR | 10-2017-0092815 | A | 14 August 2017 | KR | 10-1809599 | B1 | 15 December 2017 |
| | | | | US | 2017-0228523 | A1 | 10 August 2017 |
| | | | | WO | 2017-135496 | A1 | 10 August 2017 |
| KR | 10-2017-0017384 | A | 15 February 2017 | CN | 107949845 | A | 20 April 2018 |
| | | | | HK | 1252917 | A1 | 06 June 2019 |
| | | | | KR | 10-1817785 | B1 | 11 January 2018 |
| | | | | SG | 11201801014 | A | 28 March 2018 |
| | | | | US | 2019-0228131 | A1 | 25 July 2019 |
| | | | | WO | 2017-023148 | A1 | 09 February 2017 |
| KR | 10-2015-0070111 | A | 24 June 2015 | CA | 2883464 | A1 | 06 March 2014 |
| | | | | CN | 104968800 | A | 07 October 2015 |
| | | | | EP | 2890813 | A1 | 08 July 2015 |
| | | | | HK | 1212391 | A1 | 10 June 2016 |
| | | | | IN | 457MUN2015 | A | 04 September 2015 |
| | | | | JP | 2015-526101 | A | 10 September 2015 |
| | | | | US | 2015-0267255 | A1 | 24 September 2015 |
| | | | | WO | 2014-033455 | A1 | 06 March 2014 |
| WO | 2016-139545 | A1 | 09 September 2016 | US | 2018-0239864 | A1 | 23 August 2018 |
| US | 2014-0206006 | A1 | 24 July 2014 | CN | 102952854 | A | 06 March 2013 |
| | | | | CN | 102952854 | B | 14 January 2015 |
| | | | | EP | 2749655 | A1 | 02 July 2014 |
| | | | | EP | 2749655 | B1 | 03 July 2019 |
| | | | | HK | 1178210 | A1 | 06 September 2013 |
| | | | | WO | 2013-026411 | A1 | 28 February 2013 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020170185041
- KR 1020170144237
- KR 102018124550
- US 20070087362
- US 20060246497 A
- US 20070194225
- US 20060275779
- US 7244567 B

### Non-patent literature cited in the description

- **PARK, H., KIM et al.** *Nat Genet,* 2010, vol. 42, 400-405
- **KIDD, J.M. et al.** *Nature,* 2008, vol. 453, 56-64
- **MUJEZINOVIC F. et al.** *Obstet. Gynecol.,* 2007, vol. 110 (3), 687-94
- *ACOG Committee on Practice Bulletins,* 2007
- **LO Y. M. et al.** *The Lancet,* 1997, vol. 350 (9076), 485-7
- **GIL MM et al.** *Ultrasound Obstet. Gynecol.,* 2015, vol. 45 (3), 249-66
- **RICHARD BELLMAN.** Dynamic Programming. 2003
- **HINTON, GEOFFREY et al.** *IEEE Signal Processing Magazine,* 2012, vol. 29 (6), 82-97
- **METZKER, M.** *Nature Biotechnology Reviews,* 2010, vol. 11, 31-46
- **LASKEN R. S.** *Curr. Opin. Microbiol.,* 2007, vol. 10 (5), 510-6
- **TEWHEY R. et al.** *Nature Biotech.,* 2009, vol. 27, 1025-1031
- **PORRECA GJ et al.** *Nature Methods,* 2007, vol. 4, 931-936
- **KRISHNAKUMAR S. et al.** *Proc. Natl. Acad. Sci. USA,* 2008, vol. 105, 9296-9310
- **TURNER EH et al.** *Nature Methods,* 2009, vol. 6, 315-316
- **GNIRKE A. et al.** *Nat. Biotechnol.,* 2009, vol. 27 (2), 182-9
- **BRANTON D. et al.** *Nat. Biotechnol.,* 2008, vol. 26 (10), 1146-53
- **HANNA G. J. et al.** *J. Clin. Microbiol.,* 2000, vol. 38 (7), 2715-21
- **EDWARDS J. R. et al.** *Mut. Res.,* 2005, vol. 573 (1-2), 3-12
- **TRAPNELL C. ; SALZBERG S.L.** *Nature Biotech.,* 2009, vol. 27, 455-457
- **WARREN R. et al.** *Bioinformatics,* 2007, vol. 23, 500-501
- **BUTLER J. et al.** *Genome Res.,* 2008, vol. 18, 810-820
- **ZERBINO D.R. ; BIRNEY E.** *Genome Res.,* 2008, vol. 18, 821-829
- **HOMER N. et al.** *PLoS One,* 2009, vol. 4 (11), e7767
- **KENT W. J.** *Genome Res.,* 2002, vol. 12 (4), 656-64
- **LANGMEAD B. et al.** *Genome Biol.,* 2009, vol. 10 (3), R25
- **LI H. ; DURBIN R.** *Bioinformatics,* 2009, vol. 25, 1754-60
- **LI H. ; DURBIN R.** *Bioinformatics,* 2010, vol. 26 (5), 589-95
- **SCHATZ M. C.** *Bioinformatics,* 2009, vol. 25 (11), 1363-9
- **FAHLGREN N. et al.** *RNA,* 2009, vol. 15, 992-1002
- **SHI H. et al.** *J. Comput. Biol.,* 2010, vol. 17 (4), 603-15
- **CLEMENT N. L. et al.** *Bioinformatics,* 2010, vol. 26 (1), 38-45
- **WU T.D. ; WATANABE C.K.** *Bioinformatics,* 2005, vol. 21 (9), 1859-75
- **WU T.D. ; NACU S.** *Bioinformatics,* 2010, vol. 26 (7), 873-81
- **LI H. et al.** *Genome Res.,* 2008, vol. 18 (11), 1851-8
- **EAVES H.L. ; GAO Y.** *Bioinformatics,* 2009, vol. 25 (7), 969-70
- **CAMPAGNA D. et al.** *Bioinformatics,* 2009, vol. 25 (7), 967-8
- **PRUFER K. et al.** *Bioinformatics,* 2008, vol. 24 (13), 1530-1
- **CHEN Y. et al.** *Bioinformatics,* 2009, vol. 25 (19), 2514-2521
- **KIM Y. J. et al.** *Bioinformatics,* 2009, vol. 25 (11), 1424-5
- **DE BONA F. et al.** *Bioinformatics,* 2008, vol. 24 (16), i174
- **WEESE D. et al.** *Genome Research,* 2009, vol. 19, 1646-1654

- **SMITH A.D. et al.** *Bioinformatics,* 2009, vol. 25 (21), 2841-2
- **JIANG H. et al.** *Bioinformatics,* 2008, vol. 24, 2395-2396
- **SALMELA L.** *Bioinformatics,* 2010, vol. 26 (10), 1284-90
- **RUMBLE S.M. et al.** *PLoS Comput. Biol.,* 2009, vol. 5 (5), e1000386
- **MALHIS N. et al.** *Bioinformatics,* 2009, vol. 25 (1), 6-13
- **MULLER T. et al.** *Bioinformatics,* 2001, vol. 17 (1), S182-9
- **LI R. et al.** *Bioinformatics,* 2008, vol. 24 (5), 713-4
- **LI R. et al.** *Bioinformatics,* 2009, vol. 25 (15), 1966-7
- **ONDOV B.D. et al.** *Bioinformatics,* 2008, vol. 24 (23), 2776-7
- **NING Z. et al.** *Genome Res.,* 2001, vol. 11 (10), 1725-9
- **LUNTER G. ; GOODSON M.** *Genome Res.,* 2010